(19)

Europäisches Patentamt
European Patent Office
Office européen des brevets

(11) **EP 4 667 493 A2**

(12) **EUROPEAN PATENT APPLICATION**

(43) Date of publication:
24.12.2025  Bulletin 2025/52

(21) Application number: 25199065.1

(22) Date of filing: 25.07.2019

(51) International Patent Classification (IPC):
**C07K 16/28** (2006.01)

(52) Cooperative Patent Classification (CPC):
(C-Sets available)
**A61K 38/1774; A61K 31/337; A61K 31/407;
A61K 31/513; A61K 31/519; A61K 31/555;
A61K 33/243; A61K 39/3955; A61K 45/06;
A61P 35/00; C07K 16/2803; C07K 16/2818;**
A61K 2039/507; C07K 2317/21; C07K 2317/76
(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: 26.07.2018  US 201862703690 P
31.08.2018  US 201862725336 P

(62) Document number(s) of the earlier application(s) in
accordance with Art. 76 EPC:
**19752319.4 / 3 826 660**

(71) Applicant: **Bristol-Myers Squibb Company
Princeton, NJ 08540 (US)**

(72) Inventor: **SRIVASTAVA, Shivani
Princeton, 08540 (US)**

(74) Representative: **Mewburn Ellis LLP
Aurora Building
Counterslip
Bristol BS1 6BX (GB)**

Remarks:
•The complete document including Reference
Table(s) and the Sequence Listing(s) can be
downloaded from the EPO website
•This application was filed on 29.08.2025 as a
divisional application to the application mentioned
under INID code 62.
•Claims filed after the date of receipt of the divisional
application (Rule 68(4) EPC)

(54) **LAG-3 COMBINATION THERAPY FOR THE TREATMENT OF CANCER**

(57)    The invention provides a medicament for use in treating a tumor in a human gastric or gastro-esophageal junction cancer patient, wherein the medicament is a LAG-3 antagonist, in particular an anti-LAG-3 antibody or a soluble LAG-3, alone or in combination with a PD-1 pathway inhibitor, in particular an anti-PD-1 antibody, and optionally one or more chemotherapeutic agents.

**EP 4 667 493 A2**

(52) Cooperative Patent Classification (CPC): (Cont.)

C-Sets
**A61K 31/337, A61K 2300/00;**
**A61K 31/407, A61K 2300/00;**
**A61K 31/513, A61K 2300/00;**
**A61K 31/519, A61K 2300/00;**
**A61K 31/555, A61K 2300/00;**
**A61K 33/243, A61K 2300/00;**
**A61K 39/3955, A61K 2300/00**

**Description**

CROSS-REFERENCE TO RELATED APPLICATIONS

This application claims priority to U.S. Provisional Application Nos. 62/703,690, filed July 26, 2018 and 62/725,336, filed August 31, 2018, which are incorporated herein by reference in their entireties.

FIELD OF THE INVENTION

The invention disclosed herein relates to methods of treating a malignant tumor in a human patient with a combination of a LAG-3 inhibitor, a PD-1 pathway inhibitor, and chemotherapeutic agents.

BACKGROUND OF THE INVENTION

Lymphocyte activation gene-3 (LAG-3; CD223) is a type I transmembrane protein that is expressed on the cell surface of activated CD4+ and CD8+ T cells and subsets of NK and dendritic cells (Triebel F, et al., J. Exp. Med. 1990; 171:1393-1405; Workman C J, et al., J. Immunol. 2009; 182(4): 1885-91). LAG-3 is closely related to CD4, which is a co-receptor for T helper cell activation. Both molecules have 4 extracellular Ig-like domains and require binding to their ligand, major histocompatibility complex (MHC) class II, for their functional activity. In contrast to CD4, LAG-3 is only expressed on the cell surface of activated T cells and its cleavage from the cell surface terminates LAG-3 signaling. LAG-3 can also be found as a soluble protein but it does not bind to MHC class II and the function of soluble LAG-3 is unknown.
PD-1 is a cell surface signaling receptor that plays a critical role in the regulation of T cell activation and tolerance (Keir M E, et al., Annu Rev Immunol 2008; 26:677-704). It is a type I transmembrane protein and together with BTLA, CTLA-4, ICOS and CD28, comprise the CD28 family of T cell co-stimulatory receptors. PD-1 is primarily expressed on activated T cells, B cells, and myeloid cells (Dong H, et al., Nat Med. 1999; 5:1365-1369). It is also expressed on natural killer (NK) cells (Terme M, et al., Cancer Res 2011; 71:5393-5399). Binding of PD-1 by its ligands, PD-L1 and PD-L2, results in phosphorylation of the tyrosine residue in the proximal intracellular immune receptor tyrosine inhibitory domain, followed by recruitment of the phosphatase SHP-2, eventually resulting in down-regulation of T cell activation. One important role of PD-1 is to limit the activity of T cells in peripheral tissues at the time of an inflammatory response to infection, thus limiting the development of autoimmunity (Pardoll D M., Nat Rev Cancer 2012; 12:252-264). Evidence of this negative regulatory role comes from the finding that PD-1-deficient mice develop lupus-like autoimmune diseases including arthritis and nephritis, along with cardiomyopathy (Nishimura H, et al., Immunity, 1999; 11:141-151; and Nishimura H, et al., Science, 2001; 291:319-322). In the tumor setting, the consequence is the development of immune resistance within the tumor microenvironment. PD-1 is highly expressed on tumor-infiltrating lymphocytes, and its ligands are up-regulated on the cell surface of many different tumors (Dong H, et al., Nat Med 2002; 8:793-800). Multiple murine cancer models have demonstrated that binding of ligand to PD-1 results in immune evasion. In addition, blockade of this interaction results in anti-tumor activity (Topalian S L, et al. NEJM 2012; 366(26):2443-2454; Hamid O, et al., NEJM 2013; 369:134-144). Moreover, it has been shown that inhibition of the PD-1/PD-L1 interaction mediates potent antitumor activity in preclinical models (U.S. Pat. Nos. 8,008,449 and 7,943,743).
It is an object of the present invention to provide improved methods for treating LAG-3 positive tumors.

SUMMARY OF THE INVENTION

One aspect of the invention disclosed herein relates to a method of inhibiting the growth of a malignant tumor in a human patient, the method comprising administering to the patient an effective amount of each of: (a) a LAG-3 antagonist; (b) a PD-1 pathway inhibitor; and (c) one or more chemotherapeutic agents; wherein the patient's tumor associated immune cells express LAG-3. Another aspect of the invention relates to a method of treating cancer in a human patient, the method comprising administering to the patient an effective amount of each of: (a) a LAG-3 antagonist; (b) a PD-1 pathway inhibitor; and (c) one or more chemotherapeutic agents; wherein the patient's tumor associated immune cells express LAG-3. One aspect of the invention relates to a method of inhibiting the growth of a malignant tumor in a human patient, the method comprising administering to the patient an effective amount of each of: (a) a LAG-3 antagonist; (b) a PD-1 pathway inhibitor; and (c) one or more chemotherapeutic agents. Another aspect of the invention relates to a method of treating cancer in a human patient, the method comprising administering to the patient an effective amount of each of: (a) a LAG-3 antagonist; (b) a PD-1 pathway inhibitor; and (c) one or more chemotherapeutic agents.
In one embodiment, the malignant tumor is selected from the group consisting of a liver cancer, bone cancer, pancreatic cancer, skin cancer, oral cancer, cancer of the head or neck, breast cancer, lung cancer, including small cell and non-small cell lung cancer, cutaneous or intraocular malignant melanoma, renal cancer, uterine cancer, ovarian cancer, colorectal cancer, colon cancer, rectal cancer, cancer of the anal region, gastric cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, cancers of the childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combination thereof. In another embodiment, the malignant tumor is a gastric cancer or gastroesophageal junction cancer. In another embodiment, the gastric cancer is an adenocarcinoma, lymphoma, gastrointestinal stromal tumor, or carcinoid tumor. In another embodiment, the malignant tumor is chosen from melanoma, non-small cell lung cancer (NSCLC), human papilloma virus (HPV)-related tumor, bladder cancer, head and neck squamous cell carcinoma, renal cell cancer, and gastric adenocarcinoma.

In one embodiment of the invention, the LAG-3 antagonist is an anti-LAG-3 antibody. In another embodiment, the anti-LAG-3 antibody is a full-length antibody. In another embodiment, the antibody is a monoclonal, human, humanized, chimeric, or multispecific antibody. In another embodiment, the multispecific antibody is a dual-affinity re-targeting antibody (DART), a DVD-Ig, or bispecific antibody. In another embodiment, the antibody is a F(ab')2 fragment, a Fab' fragment, a Fab fragment, a Fv fragment, a scFv fragment, a dsFv fragment, a dAb fragment, or a single chain binding polypeptide. In another embodiment, the anti-LAG-3 antibody is BMS-986016, IMP731 (H5L7BW), MK-4280 (28G-10), REGN3767, GSK2831781, humanized BAP050, IMP-701 (LAG-525), aLAG3(0414), aLAG3(0416), Sym022, TSR-033, TSR-075, XmAb22841, BI 754111, MGD013, AVA-017, P 13B02-30, or FS-118. In another embodiment, the LAG-3 antagonist is IMP321. In another embodiment, the anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5. In another embodiment, the anti-LAG-3 antibody comprises (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; (e) a light chain variable region CDR2 comprising the sequence set forth in SEQ ID NO:11; and (f) a light chain variable region CDR3 comprising the sequence set forth in SEQ ID NO:12. In another embodiment, the anti-LAG-3 antibody comprises heavy and light chain variable regions comprising the sequences set forth in SEQ ID NOs:3 and 5, respectively. In another embodiment, the anti-LAG-3 antibody comprises heavy and light chains comprising the sequences set forth in SEQ ID NOs: 1 and 2, respectively.

In one embodiment, the PD-1 pathway inhibitor is an anti-PD-1 or an anti-PD-L1 antibody. In another embodiment, the anti-PD-1 antibody is selected from the group consisting of: nivolumab, pembrolizumab, pidilizumab, PDR001, MEDI0680, TSR-042, REGN2810, JS001, PF-06801591, BGB-A317, BI 754091, and SHR-1210. In another embodiment, the anti-PD-1 antibody comprises CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17. In another embodiment, the anti-PD-1 antibody comprises (a) a heavy chain variable region CDR1 comprising the sequence set forth in SEQ ID NO: 19; (b) a heavy chain variable region CDR2 comprising the sequence set forth in SEQ ID NO:20; (c) a heavy chain variable region CDR3 comprising the sequence set forth in SEQ ID NO:21; (d) a light chain variable region CDR1 comprising the sequence set forth in SEQ ID NO:22; (e) a light chain variable region CDR2 comprising the sequence set forth in SEQ ID NO:23; and (f) a light chain variable region CDR3 comprising the sequence set forth in SEQ ID NO:24. In another embodiment, the anti-PD-1 antibody comprises heavy and light chain variable regions comprising the sequences set forth in SEQ ID NOs: 15 and 17, respectively. In another embodiment, the anti-PD-1 antibody comprises heavy and light chains comprising the sequences as set forth in SEQ ID NOs: 13 and 14, respectively.

In one embodiment, the one or more chemotherapeutic agents are platinum compounds or fluoropyrimidines. In another embodiment, the one or more chemotherapeutic agents are oxaliplatin, cisplatin, fluorouracil, capecitabine, tegafur, gimeracil, or oteracil. In another embodiment, the one or more chemotherapeutic agents are oxaliplatin and capecitabine (XELOX). In another embodiment, the one or more chemotherapeutic agents are oxaliplatin and fluorouracil. In another embodiment, the chemotherapeutic agents further comprise a chemoprotective agent. In another embodiment, the chemoprotective agent is leucovorin. In another embodiment, the one or more chemotherapeutic agents comprise oxaliplatin, leucovorin, and fluorouracil (FOLFOX). In another embodiment, the one or more chemotherapeutic agents are oxaliplatin and tegafur/gimeracil/oteracil potassium (SOX).

In one embodiment, a fixed dose combination of the anti-LAG-3 and anti-PD-1 antibody are administered. In another embodiment, the fixed dose is determined based on the chemotherapeutic agent administered to the subject.

In one embodiment, the method comprises at least one administration cycle, wherein the cycle is a period of six weeks, and wherein for each of the at least one cycle, two doses of the anti-LAG-3 antibody are administered at a dose of 120 or 160 mg and two doses of the anti-PD-1 antibody are administered at a dose of 360 or 480 mg. In another embodiment, 120 mg of the anti-LAG-3 antibody, 360 mg of the anti-PD-1 antibody, and XELOX are administered. In another embodiment, 160 mg of the anti-LAG-3 antibody, 480 mg of the anti-PD-1 antibody, and FOLFOX are administered. In another embodiment, 120 mg of the anti-LAG-3 antibody, 360 mg of the anti-PD-1 antibody, and SOX are administered.

In one embodiment, the anti-LAG-3 and anti-PD-1 antibodies are formulated for intravenous administration. In another embodiment, the anti-LAG-3 and anti-PD-1 antibodies are formulated together. In another embodiment, the anti-LAG-3 and anti-PD-1 antibodies are formulated separately.

The present invention also related to a method of inhibiting the growth of a gastric adenocarcinoma or gastroesophageal junction adenocarcinoma in a human patient, the method comprising administering to the patient an effective amount of each of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more chemotherapeutic agents selected from the group consisting of oxaliplatin/capecitabine (XELOX), oxaliplatin/leucovorin/fluorouracil (FOLFOX), and oxaliplatin/tegafur/gimeracil/oteracil (SOX), wherein the patient's tumor-associated immune cells express LAG-3.

The present invention also relates to a method of treating gastric cancer or gastroesophageal junction cancer in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more chemotherapeutic agents selected from the group consisting of XELOX, FOLFOX, and SOX, wherein the patient's tumor-associated immune cells express LAG-3.

In one embodiment, expression of LAG-3 is assayed by RT-PCR, in situ hybridization, RNase protection, RT-PCR-based assay, immunohistochemistry, enzyme linked immuosorbent assay, in vivo imaging, or flow cytometry. In another embodiment, LAG-3 expression is assayed by immunohistochemistry.

The present invention also relates to a method of treating gastric cancer or gastroesophageal junction adenocarcinoma in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more chemotherapeutic agents. In one embodiment, the method is administered to a patient that has not received prior therapy (e.g., as a first line therapy).

The present invention also relates to a method of treating recurrent, locally advanced or metastatic gastric cancer or gastroesophageal junction adenocarcinoma in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) one or more standard-of-care therapeutic regimens, wherein the patient's tumor-associated immune cells express LAG-3.

The present invention also relates to a method of treating recurrent, locally advanced or metastatic gastric cancer or gastroesophageal junction adenocarcinoma in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more standard-of-care therapeutic regimens, wherein the patient's tumor-associated immune cells express LAG-3. One aspect of the invention relates to a method of treating recurrent, locally advanced or metastatic gastric cancer or gastroesophageal junction adenocarcinoma in a human patient, the method comprising administering to the patient an effective amount of : (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more standard-of-care therapeutic regimens. In one embodiment, the anti-LAG-3 and anti-PD-1 antibodies are administered as a fixed dose combination. In another embodiment, the one or more standard-of-care therapeutic regimens comprises administration of docetaxel, doxorubicin hydrochloride, 5-fluorouracil, mitomycin C, fluorouracil/leucovorin calcium (FU-LV), docetaxellcisplatin/fluorouracil (TPF), or capecitabine/irinotecan hydrochloride (XELIRI). In another embodiment, the method is administered to a patient that has received a prior therapy (e.g., as a second line therapy). In one embodiment, the one or more standard-of-care therapeutic regimens comprises administration of docetaxel, doxorubicin hydrochloride, 5-fluorouracil, mitomycin C, fluorouracil/leucovorin calcium (FU-LV), docetaxel/cisplatin/fluorouracil (TPF), or capecitabine/irinotecan hydrochloride (XELIRI).

The present invention also relates to a method of treating gastric cancer or gastroesophageal junction cancer in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more chemotherapeutic agents selected from the group consisting of XELOX, FOLFOX, and SOX. In embodiments, the method is administered to a patient that has not received prior therapy (e.g., first line therapy). In some embodiments, the patient has not received HER2 inhibitor therapy. In some embodiments, the method is administered to a patient who is HER2 negative. In certain embodiments, the patient has not received any prior systematic treatment. In particular embodiments, the anti-LAG-3 antibody and the anti-PD-1 antibody are administered as a fixed dose combination. In embodiments, the gastric cancer or gastroesophageal junction cancer is recurrent, locally advanced or metastatic gastric cancer or gastoesophageal adenocarcinoma.

In some embodiments of the present invention, the anti-LAG-3 antibody is Relatlimab. In some embodiments of the present invention, the anti-LAG-3 antibody comprises a serine to proline mutation at amino acid residue 228.

DETAILED DESCRIPTION OF THE INVENTION

In one aspect, the present invention relates to an improved method of treatment for malignant tumors in a human patient. In particular, the present invention shows that the administration of an anti-LAG-3 antibody in combination with an anti-PD-1 antibody, and one or more chemotherapeutic agents achieves surprisingly improved treatment outcomes in a patient population having a LAG-3 positive malignant tumor, than in a population comprising patients having both LAG-3 positive and LAG-3 negative tumors. Accordingly, in one aspect, the invention described herein relates to a method of treating a LAG-3 positive malignant tumor (e.g., gastric adenocarcinoma, or a gastroesophageal junction adenocarcinoma) by administering a combination of a LAG-3 inhibitor (e.g., anti-LAG-3 antibody) and a PD-1 pathway inhibitor (e.g., an anti-PD-1 antibody), and one or more chemotherapeutic agents.

## 1. Definitions

In order that the present disclosure may be more readily understood, certain terms are first defined. As used in this application, except as otherwise expressly provided herein, each of the following terms shall have the meaning set forth below. Additional definitions are set forth throughout the application.

An "antibody" (Ab) shall include, without limitation, a glycoprotein immunoglobulin which binds specifically to an antigen and comprises at least two heavy (H) chains and two light (L) chains interconnected by disulfide bonds. Each H chain comprises a heavy chain variable region (abbreviated herein as $V_H$) and a heavy chain constant region. The heavy chain constant region comprises three constant domains, $C_{H1}$, $C_{H2}$ and $C_{H3}$. Each light chain comprises a light chain variable region (abbreviated herein as $V_L$) and a light chain constant region. The light chain constant region is comprises one constant domain, $C_L$. The $V_H$ and $V_L$ regions can be further subdivided into regions of hypervariability, termed complementarity determining regions (CDRs), interspersed with regions that are more conserved, termed framework regions (FR). Each $V_H$ and $V_L$ comprises three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. A heavy chain may have the C-terminal lysine or not. Unless specified otherwise herein, the amino acids in the variable regions are numbered using the Kabat numbering system and those in the constant regions are numbered using the EU system. In one embodiment, an antibody is an intact antibody.

An immunoglobulin may derive from any of the commonly known isotypes, including but not limited to IgA, secretory IgA, IgG and IgM. IgG subclasses are also well known to those in the art and include but are not limited to human IgG1, IgG2, IgG3 and IgG4. "Isotype" refers to the antibody class or subclass (e.g., IgM or IgG1) that is encoded by the heavy chain constant region genes. The term "antibody" includes, by way of example, monoclonal and polyclonal antibodies; chimeric and humanized antibodies; human or nonhuman antibodies; wholly synthetic antibodies; and single chain antibodies. A nonhuman antibody may be humanized by recombinant methods to reduce its immunogenicity in man. Where not expressly stated, and unless the context indicates otherwise, the term "antibody" includes monospecific, bispecific, or multi-specific antibodies, as well as a single chain antibody. In embodiments, the antibody is a bispecific antibody. In other embodiments, the antibody is a monospecific antibody. In one aspect, the constant region isotype is IgG4 with a mutation at amino acid residue 228, e.g., S228P.

As used herein, an "IgG antibody" has the structure of a naturally occurring IgG antibody, *i.e.,* it has the same number of heavy and light chains and disulfide bonds as a naturally occurring IgG antibody of the same subclass. For example, an anti-LAG-3 IgG1, IgG2, IgG3 or IgG4 antibody consists of two heavy chains (HCs) and two light chains (LCs), wherein the two heavy chains and light chains are linked by the same number and location of disulfide bridges that occur in naturally occurring IgG1, IgG2, IgG3 and IgG4 antibodies, respectively (unless the antibody has been mutated to modify the disulfide bonds)

An "isolated antibody" refers to an antibody that is substantially free of other antibodies having different antigenic specificities (*e.g.*, an isolated antibody that binds specifically to LAG-3 is substantially free of antibodies that bind specifically to antigens other than LAG-3). An isolated antibody that binds specifically to LAG-3 may, however, have cross-reactivity to other antigens, such as LAG-3 molecules from different species. Moreover, an isolated antibody may be substantially free of other cellular material and/or chemicals.

The antibody may be an antibody that has been altered (*e.g.,* by mutation, deletion, substitution, conjugation to a non-antibody moiety). For example, an antibody may include one or more variant amino acids (compared to a naturally occurring antibody) which change a property (*e.g.,* a functional property) of the antibody. For example, numerous such alterations are known in the art which affect, e.g., half-life, effector function, and/or immune responses to the antibody in a patient. The term antibody also includes artificial polypeptide constructs which comprise at least one antibody-derived antigen binding site.

The term "monoclonal antibody" ("mAb") refers to a non-naturally occurring preparation of antibody molecules of single molecular composition, *i.e.,* antibody molecules whose primary sequences are essentially identical, and which exhibits a single binding specificity and affinity for a particular epitope. A mAb is an example of an isolated antibody. MAbs may be produced by hybridoma, recombinant, transgenic or other techniques known to those skilled in the art.

A "human" antibody (HuMAb) refers to an antibody having variable regions in which both the framework and CDR regions are derived from human germline immunoglobulin sequences. Furthermore, if the antibody contains a constant region, the constant region is also derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo*). However, the term "human antibody," as used herein, is not intended to include antibodies in which CDR sequences derived from the germline of another mammalian species, such as a mouse, have been grafted onto human framework sequences. The terms "human" antibodies and "fully human" antibodies and are used synonymously.

A "humanized antibody" refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody.

An "anti-antigen" antibody refers to an antibody that binds specifically to the antigen. For example, an anti-LAG-3 antibody binds specifically to LAG-3.

An "antigen-binding portion" of an antibody (also called an "antigen-binding fragment") refers to one or more fragments of an antibody that retain the ability to bind specifically to the antigen bound by the whole antibody. It has been shown that the antigen-binding function of an antibody can be performed by fragments or portions of a full-length antibody. Examples of binding fragments encompassed within the term "antigen-binding portion" or "antigen-binding fragment" of an antibody, e.g., an anti-LAG-3 antibody described herein, include:

(1) a Fab fragment (fragment from papain cleavage) or a similar monovalent fragment consisting of the VL, VH, LC and CH1 domains;

(2) a F(ab')2 fragment (fragment from pepsin cleavage) or a similar bivalent fragment comprising two Fab fragments linked by a disulfide bridge at the hinge region;

(3) a Fd fragment consisting of the VH and CH1 domains;

(4) a Fv fragment consisting of the VL and VH domains of a single arm of an antibody,

(5) a single domain antibody (dAb) fragment (Ward et al., (1989) Nature 341:544-46), which consists of a VH domain;

(6) a bi-single domain antibody which consists of two VH domains linked by a hinge (dual-affinity re-targeting antibodies (DARTs));

(7) a dual variable domain immunoglobulin;

(8) an isolated complementarity determining region (CDR); and

(9) a combination of two or more isolated CDRs, which can optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); *see, e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" or "antigen-binding fragment" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins. In some embodiments, an antibody is an antigen-binding fragment.

The term "LAG-3" refers to Lymphocyte Activation Gene-3. The term "LAG-3" includes variants, isoforms, homologs, orthologs and paralogs. For example, antibodies specific for a human LAG-3 protein may, in certain cases, cross-react with a LAG-3 protein from a species other than human. In other embodiments, the antibodies specific for a human LAG-3 protein may be completely specific for the human LAG-3 protein and may not exhibit species or other types of cross-reactivity, or may cross-react with LAG-3 from certain other species, but not all other species (e.g., cross-react with monkey LAG-3 but not mouse LAG-3). The term "human LAG-3" refers to human sequence LAG-3, such as the complete amino acid sequence of human LAG-3 having GenBank Accession No. NP_002277. The term "mouse LAG-3" refers to mouse sequence LAG-3, such as the complete amino acid sequence of mouse LAG-3 having GenBank Accession No. NP_032505. LAG-3 is also known in the art as, for example, CD223. The human LAG-3 sequence may differ from human LAG-3 of GenBank Accession No. NP_002277 by having, e.g., conserved mutations or mutations in non-conserved regions and the LAG-3 has substantially the same biological function as the human LAG-3 of GenBank Accession No. NP_002277. For example, a biological function of human LAG-3 is having an epitope in the extracellular domain of LAG-3 that is specifically bound by an antibody of the instant disclosure or a biological function of human LAG-3 is binding to MHC Class II molecules.

A particular human LAG-3 sequence will generally be at least 90% identical in amino acid sequence to human LAG-3 of GenBank Accession No. NP_002277 and contains amino acid residues that identify the amino acid sequence as being human when compared to LAG-3 amino acid sequences of other species (e.g., murine). In certain cases, a human LAG-3 can be at least 95%, or even at least 96%, 97%, 98%, or 99% identical in amino acid sequence to LAG-3 of GenBank Accession No. NP_002277. In certain embodiments, a human LAG-3 sequence will display no more than 10 amino acid differences from the LAG-3 sequence of GenBank Accession No. NP_002277. In certain embodiments, the human LAG-3 can display no more than 5, or even no more than 4, 3, 2, or 1 amino acid difference from the LAG-3 sequence of GenBank Accession No. NP_002277. Percent identity can be determined as described herein.

As used herein, the terms "Programmed Death 1," "Programmed Cell Death 1," "Protein PD-1," "PD-1," "PD1," "PDCD1," "hPD-1" and "hPD-I" are used interchangeably, and include variants, isoforms, species homologs of human PD-1, and analogs having at least one common epitope with PD-1. The complete PD-1 sequence can be found under GenBank Accession No. U64863.

The protein Programmed Death 1 (PD-1) is an inhibitory member of the CD28 family of receptors, that also includes CD28, CTLA-4, ICOS and BTLA. PD-1 is expressed on activated B cells, T cells, and myeloid cells (Agata *et al., supra;* Okazaki et al. (2002) Curr. Opin. Immunol. 14: 391779-82; Bennett et al. (2003) J Immunol 170:711-8). The initial members of the family, CD28 and ICOS, were discovered by functional effects on augmenting T cell proliferation following the addition of monoclonal antibodies (Hutloff et al. Nature (1999); 397:263-266; Hansen et al. Immunogenics (1980); 10:247-260). PD-1 was discovered through screening for differential expression in apoptotic cells (Ishida et al. EMBO J (1992); 11:3887-95). The other members of the family, CTLA-4 and BTLA, were discovered through screening for differential expression in cytotoxic T lymphocytes and TH1 cells, respectively. CD28, ICOS and CTLA-4 all have an unpaired cysteine residue allowing for homodimerization. In contrast, PD-1 is suggested to exist as a monomer, lacking the unpaired cysteine residue characteristic in other CD28 family members.

The PD-1 gene is a 55 kDa type I transmembrane protein that is part of the Ig gene superfamily (Agata et al. (1996) Int Immunol 8:765-72). PD-1 contains a membrane proximal immunoreceptor tyrosine inhibitory motif (ITIM) and a membrane distal tyrosine-based switch motif (ITSM) (Thomas, M. L. (1995) J Exp Med 181:1953-6; Vivier, E and Daeron, M (1997) Immunol Today 18:286-91). Although structurally similar to CTLA-4, PD-1 lacks the MYPPPY motif (SEQ ID NO: 32) that is critical for B7-1 and B7-2 binding. Two ligands for PD-1 have been identified, PD-L1 and PD-L2, that have been shown to downregulate T cell activation upon binding to PD-1 (Freeman et al. (2000) J Exp Med 192:1027-34; Latchman et al. (2001) Nat Immunol 2:261-8; Carter et al. (2002) Eur J Immunol 32:634-43). Both PD-L1 and PD-L2 are B7 homologs that bind to PD-1, but do not bind to other CD28 family members. PD-L1 is abundant in a variety of human cancers (Dong et al. (2002) Nat. Med. 8:787-9). The interaction between PD-1 and PD-L1 results in a decrease in tumor infiltrating lymphocytes, a decrease in T-cell receptor mediated proliferation, and immune evasion by the cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1, and the effect is additive when the interaction of PD-1 with PD-L2 is blocked as well (Iwai et al. (2002) Proc. Nat'l. Acad. Sci. USA 99:12293-7; Brown et al. (2003) J. Immunol. 170:1257-66).

Consistent with PD-1 being an inhibitory member of the CD28 family, PD-1 deficient animals develop various autoimmune phenotypes, including autoimmune cardiomyopathy and a lupus-like syndrome with arthritis and nephritis (Nishimura et al. (1999) Immunity 11:141-51; Nishimura et al. (2001) Science 291:319-22). Additionally, PD-1 has been found to play a role in autoimmune encephalomyelitis, systemic lupus erythematosus, graft-versus-host disease (GVHD), type I diabetes, and rheumatoid arthritis (Salama et al. (2003) J Exp Med 198:71-78; Prokunina and Alarcon-Riquelme (2004) Hum Mol Genet 13:R143; Nielsen et al. (2004) Lupus 13:510). In a murine B cell tumor line, the ITSM of PD-1 was shown to be essential to block BCR-mediated Ca.sup.2+-flux and tyrosine phosphorylation of downstream effector molecules (Okazaki et al. (2001) PNAS 98: 13866-71).

"Programmed Death Ligand-1 (PD-L1)" is one of two cell surface glycoprotein ligands for PD-1 (the other being PD-L2) that downregulate T cell activation and cytokine secretion upon binding to PD-1. The term "PD-L1" as used herein includes human PD-L1 (hPD-L1), variants, isoforms, and species homologs of hPD-L1, and 5 analogs having at least one common epitope with hPD-L1. The complete hPD-L1 sequence can be found under GenBank Accession No. Q9NZQ7.

The terms "Programmed Death Ligand-2" and "PD-L2" as used herein include human PD-L2 (hPD-L2), variants, isoforms, and species homologs of hPD-L2, and analogs having at least one common epitope with hPD-L2. The complete hPD-L2 sequence can be found under GenBank Accession No. Q9BQ51.

A "patient" as used herein includes any patient who is afflicted with a cancer (e.g., gastric or gastroesophageal cancer). The terms "subject" and "patient" are used interchangeably herein.

"Administering" refers to the physical introduction of a composition comprising a therapeutic agent to a subject, using any of the various methods and delivery systems known to those skilled in the art. Routes of administration for the formulations disclosed herein include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as in vivo electroporation. In some embodiments, the formulation is administered via a non-parenteral route, in some embodiments, orally. Other non-parenteral routes include a topical, epidermal or mucosal route of administration, for example, intranasally, vaginally, rectally, sublingually or topically. Administering can also be performed, for example, once, a plurality of times, and/or over one or more extended periods.

"Treatment" or "therapy" of a subject refers to any type of intervention or process performed on, or the administration of an active agent to, the subject with the objective of reversing, alleviating, ameliorating, inhibiting, slowing down progression, development, severity or recurrence of a symptom, complication or condition, or biochemical indicia associated with a disease. Response Evaluation Criteria In Solid Tumors (RECIST) is a measure for treatment efficacy and are established rules that define when tumors respond, stabilize, or progress during treatment. RECIST 1.1 is the current guideline to solid tumor measurement and definitions for objective assessment of change in tumor size for use in adult and pediatric cancer clinical trials.

As used herein, "effective treatment" refers to treatment producing a beneficial effect, e.g., amelioration of at least one symptom of a disease or disorder. A beneficial effect can take the form of an improvement over baseline, i.e., an improvement over a measurement or observation made prior to initiation of therapy according to the method. A beneficial effect can also take the form of arresting, slowing, retarding, or stabilizing of a deleterious progression of a marker of solid tumor. Effective treatment may refer to alleviation of at least one symptom of a solid tumor. Such effective treatment may, e.g., reduce patient pain, reduce the size and/or number of lesions, may reduce or prevent metastasis of a tumor, and/or may slow tumor growth.

The term "effective amount" refers to an amount of an agent that provides the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In reference to solid tumors, an effective amount comprises an amount sufficient to cause a tumor to shrink and/or to decrease the growth rate of the tumor (such as to suppress tumor growth) or to delay other unwanted cell proliferation. In some embodiments, an effective amount is an amount sufficient to prevent or delay tumor recurrence. An effective amount can be administered in one or more administrations. The effective amount of the drug or composition may: (i) reduce the number of cancer cells; (ii) reduce tumor size; (iii) inhibit, retard, slow to some extent and may stop cancer cell infiltration into peripheral organs; (iv) inhibit (i.e., slow to some extent and may stop tumor metastasis; (v) inhibit tumor growth; (vi) prevent or delay occurrence and/or recurrence of tumor; and/or (vii) relieve to some extent one or more of the symptoms associated with the cancer. In one example, an "effective amount" is the amount of anti-LAG-3 antibody, the amount of anti-PD-1 antibody, and the amount of chemotherapeutic agents, in combination, clinically proven to affect a significant decrease in cancer or slowing of progression of cancer, such as an advanced solid tumor. As used herein, the terms "fixed dose", "flat dose" and "flat-fixed dose" are used interchangeably and refer to a dose that is administered to a patient without regard for the weight or body surface area (BSA) of the patient. The fixed or flat dose is therefore not provided as a mg/kg dose, but rather as an absolute amount of the agent (e.g., the anti-LAG-3 antibody and/or anti-PD-1 antibody). For example, a 60 kg person and a 100 kg person would receive the same dose of the composition (e.g., 360 mg of an anti-PD-1 antibody and 120 mg of an anti- LAG-3 antibody in a single fixed dosing formulation vial containing both 360 mg of an anti-PD-1 antibody and 120 mg of an anti- LAG-3 antibody (or two fixed dosing formulation vials containing 180 mg of an anti-PD-1 antibody and 60 mg of an anti- LAG-3 antibody, etc.)).

The use of the term "fixed dose combination" with regard to a composition of the invention means that two or more different antibodies in a single composition are present in the composition in particular (fixed) ratios with each other. In some embodiments, the fixed dose is based on the weight (e.g., mg) of the antibodies. In certain embodiments, the fixed dose is based on the concentration (e.g., mg/ml) of the antibodies. In some embodiments, the ratio is at least about 1:1, about 1:2, about 1:3, about 1:4, about 1:5, about 1:6, about 1:7, about 1:8, about 1:9, about 1:10, about 1:15, about 1:20, about 1:30, about 1:40, about 1:50, about 1:60, about 1:70, about 1:80, about 1:90, about 1:100, about 1:120, about 1:140, about 1:160, about 1:180, about 1:200, about 200:1, about 180:1, about 160:1, about 140:1, about 120:1, about 100:1, about 90:1, about 80:1, about 70:1, about 60:1, about 50:1, about 40:1, about 30:1, about 20:1, about 15:1, about 10:1, about 9:1, about 8:1, about 7:1, about 6:1, about 5:1, about 4:1, about 3:1, or about 2:1 mg first antibody to mg second antibody. For example, the 3:1 ratio of a first antibody and a second antibody can mean that a vial can contain about 240 mg of the first antibody and 80 mg of the second antibody or about 3 mg/ml of the first antibody and 1 mg/ml of the second antibody.

The term "weight based dose" as referred to herein means that a dose that is administered to a patient is calculated based on the weight of the patient. For example, when a patient with 60 kg body weight requires 3 mg/kg of an anti-LAG-3 antibody in combination with 3 mg/kg of an anti-PD-1 antibody, one can draw the appropriate amounts of the anti-LAG-3 antibody (i.e., 180 mg) and the anti-PD-1 antibody (i.e., 180 mg) at once from a 1:1 ratio fixed dose combination of an anti-LAG3 antibody and an anti-PD-1 antibody.

The term "progression-free survival," which can be abbreviated as PFS, as used herein refers to the length of time during and after the treatment of a solid tumor (i.e., melanoma) that a patient lives with the disease but it does not get worse.

"Dosing interval," as used herein, means the amount of time that elapses between multiple doses of a formulation disclosed herein being administered to a subject. Dosing interval can thus be indicated as ranges.

The term "dosing frequency" as used herein refers to the frequency of administering doses of a formulation disclosed herein in a given time. Dosing frequency can be indicated as the number of doses per a given time, e.g., once a week or once in two weeks.

The terms "about once a week," "once about every week," "once about every two weeks," or any other similar dosing interval terms as used herein means approximate number, and "about once a week" or "once about every week" can include every seven days ± two days, i.e., every five days to every nine days. The dosing frequency of "once a week" thus can be every five days, every six days, every seven days, every eight days, or every nine days. "Once about every two weeks" can include every fourteen days ± three days, i.e., every eleven days to every seventeen days. Similar approximations apply, for example, to once about every three weeks, once about every four weeks, once about every five weeks, once about every six weeks and once about every twelve weeks. In some embodiments, a dosing interval of once about every six weeks or once about every twelve weeks means that the first dose can be administered any day in the first week, and then the next dose can be administered any day in the sixth or twelfth week, respectively. In other embodiments, a dosing interval of once about every six weeks or once about every twelve weeks means that the first dose is administered on a particular day of the first week (e.g., Monday) and then the next dose is administered on the same day of the sixth or twelfth weeks (i.e., Monday), respectively.

A "cancer" refers a broad group of various diseases characterized by the uncontrolled growth of abnormal cells in the body. Unregulated cell division and growth results in the formation of malignant tumors that invade neighboring tissues and may also metastasize to distant parts of the body through the lymphatic system or bloodstream. A "cancer" or "cancer tissue" can include a tumor. "Gastric cancer" and "stomach cancer" are used interchangeably herein. As used herein, "gastric cancer" can develop in any part of the stomach, and may spread throughout the stomach and to other organs. It may grow along the stomach wall into the esophagus or small intestine. The cancer may also extend through the stomach wall and spread to nearby lymph nodes and organs, such as the liver, pancreas and colon. It may spread to distant organs, such as the lungs, the lymph nodes above the collarbone and to a woman's ovaries. The different types of gastric cancer include adenocarcinomas, lymphoma, gastrointestinal stromal tumors (GISTs) and carcinoid tumors.

The term "tumor" as used herein refers to any mass of tissue that results from excessive cell growth or proliferation, either benign (non-cancerous) or malignant (cancerous), including pre-cancerous lesions.

The term "LAG-3 positive" or "LAG-3 expression positive," relating to LAG-3 expression, refers to the proportion of cells in a test tissue sample comprising tumor cells and tumor-infiltrating inflammatory cells above which the tissue sample is scored as expressing LAG-3. In some embodiments, for LAG-3 expression assayed by immunohistochemistry (IHC), the LAG-3 positive tumor or LAG-3 expression positive tumor means that at least about 0.01%, at least about 0.5%, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 40%, at least about 50%, at least about 60%, at least about 70%, at least about 80%, at least about 90%, or 100% of the total number of cells express LAG-3. In other embodiments, for LAG-3 expression assayed by immunohistochemistry (IHC) or flow cytometry, the LAG-3 positive tumor or LAG-3 expression positive tumor means that at least about 0.01%, at least about 0.5%, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 40%, at least about 50%, at least about 60%, at least about 70%, at least about 80%, at least about 90%, or 100% of the total number of tumor-associated inflammatory cells (e.g., T cells, CD8+ T cells, CD4+ T cells, FOXP3+ cells) express LAG-3. LAG-3 positive tumor or LAG-3 expression positive tumor can also be expressed herein as tumor expressing LAG-3. In some embodiments, the LAG-3 positive tumor or LAG-3 expression positive tumor means that at least about 0.1% to at least about 20% of the total number of cells express LAG-3. In some embodiments, a LAG-3 positive tumor or LAG-3 expression positive tumor means that at least about 0.1% to at least about 20% of the total number of tumor-associated inflammatory cells (e.g., T cells, CD8+ T cells, CD4+ T cells, FOXP3+ cells) express LAG-3. In certain embodiments, a LAG-3 positive tumor or LAG-3 expression positive tumor means that at least about 0.1% to at least about 10% of the total number of cells express LAG-3. In certain embodiments, a LAG-3 positive tumor or LAG-3 expression positive tumor means that at least about 0.1% to at least about 10% of the total number of tumor-infiltrating inflammatory cells (e.g., T cells, CD8+ T cells, CD4+ T cells, FOXP3+ cells) express LAG-3. In some embodiments, a LAG-3 positive or LAG-3 expression positive tumor means that at least about 1% of the total number of cells express LAG-3 on the cell surface. In some embodiments, a LAG-3 positive or LAG-3 expression positive tumor means that at least about 1% of the total number of tumor-infiltrating inflammatory cells (e.g., T cells, CD8+ T cells, CD4+ T cells, FOXP3+ cells) express LAG-3 on the cell surface. In other embodiments, a LAG-3 positive or LAG-3 expression positive tumor means that at least about 5% of the total number of cells express LAG-3 on the cell surface. In other embodiments, a LAG-3 positive or LAG-3 expression positive tumor means that at least about 5% of the total number of tumor-infiltrating inflammatory cells (e.g., T cells, CD8+ T cells, CD4+ T cells, FOXP3+ cells) express LAG-3 on the cell surface. In one particular embodiment, LAG-3 positive or LAG-3 expression positive tumor means that at least about 1%, or in the range of 1- 5% of the total number of cells express LAG-3 on the cell surface. In one particular embodiment, LAG-3 positive or LAG-3 expression positive tumor means that at least about 1%, or in the range of 1- 5% of the total number of tumor-infiltrating inflammatory cells (e.g., T cells, CD8+ T cells, CD4+ T cells, FOXP3+ cells) express LAG-3 on the cell surface.

"LAG-3 negative" or "LAG-3 expression negative," refers to the proportion of cells in a test tissue sample comprising tumor cells and tumor- infiltrating inflammatory cells that are not LAG-3 positive or LAG-3 expression positive.

The term "PD-L1 positive" or "PD-L1 expression positive," relating to cell surface PD-L1 expression, refers to the proportion of cells in a test tissue sample comprising tumor cells and tumor- infiltrating inflammatory cells above which the sample is scored as expressing cell surface PD-L1. For cell surface expression assayed by immunohistochemistry (IHC), e.g., with the mAb 28- 8, the PD-L1 positive tumor or PD-L1 expression positive tumor means that at least about 0.01%, at least about 0.5%, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, or at least about 30% of the total number of cells express PD-L1. PD-L1 positive tumor or PD-L1 expression positive tumor can also be expressed herein as tumor expressing PD-L1. In other embodiments, the PD-L1 positive tumor or PD-L1 expression positive tumor means that at least about 0.1% to at least about 20% of the total number of cells express PD-L1. In certain embodiments, the PD-L1 positive tumor or PD-L1 expression positive tumor means that at least about 0.1% to at least about 10% of the total number of cells express PD-L1. In some embodiments, the PD-L1 positive or PD-L1 expression positive tumor means that at least about 1% of the total number of cells express PD-L1 on the cell surface. In other embodiments, the PD-L1 positive or PD-L1 expression positive tumor means that at least about 5% of the total number of cells express PD-L1 on the cell surface. In one particular embodiment, PD-L1 positive or PD-L1 expression positive tumor means that at least about 1%, or in the range of 1- 5% of the total number of cells express PD-L1 on the cell surface.

The term "PD-L1 negative" or "PD-L1 expression negative," relating to cell surface PD-L1 expression, refers to the proportion of cells in a test tissue sample comprising tumor cells and tumor- infiltrating inflammatory cells that are not PD-L1 positive or PD-L1 expression positive.

The term "evaluable PD-L1 expression status," relates to a measurable expression level of PD-L1, generally $\geq$ 5% or < 5%.

The term "tumor mutation burden" (TMB) as used herein refers to the number of somatic mutations in a tumor's genome and/or the number of somatic mutations per area of the tumor's genome. Germline (inherited) variants are excluded when determining TMB, because the immune system has a higher likelihood of recognizing these as self. Tumor mutation burden (TMB) can also be used interchangeably with "tumor mutation load," "tumor mutational burden," or "tumor mutational load."

TMB is a genetic analysis of a tumor's genome and, thus, can be measured by applying sequencing methods well known to those of skill in the art. The tumor DNA can be compared with DNA from patient-matched normal tissue to eliminate germline mutations or polymorphisms.

In some embodiments, TMB is determined by sequencing tumor DNA using a high-throughput sequence technique, e.g., next-generation sequencing (NGS) or an NGS-based method. In some embodiments, the NGS-based method is selected from whole genome sequencing (WGS), whole exome sequencing (WES), or comprehensive genomic profiling (CGP) of cancer gene panels such as FOUNDATIONONE® CDX™ and MSK-IMPACT clinical tests. In some embodiments, TMB, as used herein, refers to the number of somatic mutations per megabase (Mb) of DNA sequenced. In one embodiment, TMB is measured using the total number of nonsynonymous mutations, e.g., missense mutation *(i.e.,* changing a particular amino acid in the protein) and/or nonsense (causing premature termination and thus truncation of the protein sequence), identified by normalizing matched tumor with germline samples to exclude any inherited germline genetic alterations. In another embodiment, TMB is measured using the total number of missense mutations in a tumor. In order to measure TMB, a sufficient amount of sample is required. In one embodiment, tissue sample (for example, a minimum of 10 slides) is used for evaluation. In some embodiments, TMB is expressed as NsMs per megabase (NsM/Mb). 1 megabase represents 1 million bases.

The TMB status can be a numerical value or a relative value, e.g., high, medium, or low; within the highest fractile, or within the top tertile, of a reference set.

The term "high TMB" as used herein refers to a number of somatic mutations in a tumor's genome that is above a number of somatic mutations that is normal or average. In some embodiments, a TMB has a score of at least 210, at least 215, at least 220, at least 225, at least 230, at least 235, at least 240, at least 245, at least 250, at least 255, at least 260, at least 265, at least 270, at least 275, at least 280, at least 285, at least 290, at least 295, at least 300, at least 305, at least 310, at least 315, at least 320, at least 325, at least 330, at least 335, at least 340, at least 345, at least 350, at least 355, at least 360, at least 365, at least 370, at least 375, at least 380, at least 385, at least 390, at least 395, at least 400, at least 405, at least 410, at least 415, at least 420, at least 425, at least 430, at least 435, at least 440, at least 445, at least 450, at least 455, at least 460, at least 465, at least 470, at least 475, at least 480, at least 485, at least 490, at least 495, or at least 500; in other embodiments a high TMB has a score of at least at least 221, at least 222, at least 223, at least 224, at least 225, at least 226, at least 227, at least 228, at least 229, at least 230, at least 231, at least 232, at least 233, at least 234, at least 235, at least 236, at least 237, at least 238, at least 239, at least 240, at least 241, at least 242, at least 243, at least 244, at least 245, at least 246, at least 247, at least 248, at least 249, or at least 250; and, in a particular embodiment, a high TMB has a score of at least 243. In other embodiments, a "high TMB" refers to a TMB within the highest fractile of the reference TMB value. For example, all subject's with evaluable TMB data are grouped according to fractile distribution of TMB, *i.e.,* subjects are rank ordered from highest to lowest number of genetic alterations and divided into a defined number of groups. In one embodiment, all subjects with evaluable TMB data are rank ordered and divided into thirds and a "high TMB" is within the top tertile of the reference TMB value. In a particular embodiment, the tertile boundaries are 0 < 100 genetic alterations; 100 to 243 genetic alterations; and > 243 genetic alterations. It should be understood that, once rank ordered, subjects with evaluable TMB data can be divided into any number of groups, e.g., quartiles, quintiles, etc. In some embodiments, a "high TMB" refers to a TMB of at least about 20 mutations/tumor, at least about 25 mutations/tumor, at least about 30 mutations/tumor, at least about 35 mutations/tumor, at least about 40 mutations/tumor, at least about 45 mutations/tumor, at least about 50 mutations/tumor, at least about 55 mutations/tumor, at least about 60 mutations/tumor, at least about 65 mutations/tumor, at least about 70 mutations/tumor, at least about 75 mutations/tumor, at least about 80 mutations/tumor, at least about 85 mutations/tumor, at least about 90 mutations/tumor, at least about 95 mutations/tumor, or at least about 100 mutations/tumor. In some embodiments, a "high TMB" refers to a TMB of at least about 105 mutations/tumor, at least about 110 mutations/tumor, at least about 115 mutations/tumor, at least about 120 mutations/tumor, at least about 125 mutations/tumor, at least about 130 mutations/tumor, at least about 135 mutations/tumor, at least about 140 mutations/tumor, at least about 145 mutations/tumor, at least about 150 mutations/tumor, at least about 175 mutations/tumor, or at least about 200 mutations/tumor. In certain embodiments, a tumor having a high TMB has at least about 100 mutations/tumor.

The "high TMB" can also be referred to as the number of mutations per megabase of genome sequenced, *e.g.,* as measured by a mutation assay, *e.g.,* FOUNDATIONONE® CDX™ assay. In one embodiment, the high TMB refers to at least about 9, at least about 10, at least about 11, at least 12, at least 13, at least about 14, at least about 15, at least about 16, at least about 17, at least about 18, at least about 19, or at least about 20 mutations per megabase of genome as measured by a FOUNDATIONONE® CDX™ assay. In a particular embodiment, the "high TMB" refers to at least 10 mutations per megabase of genome sequenced by a FOUNDATIONONE® CDX™ assay.

As used herein, the term "medium TMB" refers to a number of somatic mutations in a tumor's genome that is at or around a number of somatic mutations that is normal or average and the term "low TMB" refers to a number of somatic mutations in a tumor's genome that is below a number of somatic mutations that is normal or average. In a particular embodiment, a "high TMB" has a score of at least 243, a "medium TMB" has a score of between 100 and 242, and a "low TMB" has a score of less than 100 (or between 0 and 100). The "medium or low TMB" refers to less than 9 mutations per megabase of genome sequenced, e.g., as measured by a FOUNDATIONONE® CDX™ assay.

Microsatellite instability is the condition of genetic hypermutability that results from impaired DNA mismatch repair (MMR). The presence of MSI represents phenotypic evidence that MMR is not functioning normally. In most cases, the genetic basis for instability in MSI tumors is an inherited germline alteration in any one of the five human MMR genes: MSH2, MLH1, MSH6, PMS2, and PMS1. In certain embodiments, the subject receiving tumor treatment has a high degree of microsatellite instability (MSI-H) and has at least one mutation in genes MSH2, MLH1, MSH6, PMS2, or PMS1. In other embodiments, subjects receiving tumor treatment within a control group have no microsatellite instability (MSS or MSI stable) and has no mutation in genes MSH2, MLH1, MSH6, PMS2, and PMS1

An "immune response" refers to the action of a cell of the immune system (for example, T lymphocytes, B lymphocytes, natural killer (NK) cells, macrophages, eosinophils, mast cells, dendritic cells and neutrophils) and soluble macromolecules produced by any of these cells or the liver (including antibodies, cytokines, and complement) that results in selective targeting, binding to, damage to, destruction of, and/or elimination from a vertebrate's body of invading pathogens, cells or tissues infected with pathogens, cancerous or other abnormal cells, or, in cases of autoimmunity or pathological inflammation, normal human cells or tissues.

A "tumor-infiltrating inflammatory cell" or "tumor-associated inflammatory cell" is any type of cell that typically participates in an inflammatory response in a subject and which infiltrates tumor tissue. Such cells include tumor-infiltrating lymphocytes (TILs), macrophages, monocytes, eosinophils, histiocytes and dendritic cells.

The use of the alternative (e.g., "or") should be understood to mean either one, both, or any combination thereof of the alternatives. As used herein, the indefinite articles "a" or "an" should be understood to refer to "one or more" of any recited or enumerated component.

The term "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

The terms "about" or "comprising essentially of" refer to a value or composition that is within an acceptable error range for the particular value or composition as determined by one of ordinary skill in the art, which will depend in part on how the value or composition is measured or determined, *i.e.,* the limitations of the measurement system. For example, "about" or "comprising essentially of" can mean within 1 or more than 1 standard deviation per the practice in the art. Alternatively, "about" or "comprising essentially of" can mean a range of up to 10% or 20% (*i.e.,* ±10% or ±20%). For example, about 3mg can include any number between 2.7 mg and 3.3 mg (for 10%) or between 2.4 mg and 3.6 mg (for 20%). Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

As described herein, any concentration range, percentage range, ratio range or integer range is to be understood to include the value of any integer within the recited range and, when appropriate, fractions thereof (such as one-tenth and one-hundredth of an integer), unless otherwise indicated.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 5th ed., 2013, Academic Press; and the Oxford Dictionary Of Biochemistry And Molecular Biology, 2006, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. The headings provided herein are not limitations of the various aspects of the disclosure, which can be had by reference to the specification as a whole. Accordingly, the terms defined immediately below are more fully defined by reference to the specification in its entirety.

Various aspects of the invention are described in further detail in the following subsections.

## 2. Methods of the Invention

In one aspect, the present invention is directed to a method for treating a LAG-3-positive malignant tumor (e.g., gastric cancer or gastroesophageal junction cancer) in a subject in need thereof. A combination therapy of a LAG-3 inhibitor (e.g., anti-LAG-3 antibody), a PD-1 pathway inhibitor (e.g., anti-PD-1 antibody), and one or more chemotherapeutic agents results in better therapeutic outcomes (e.g., objective response rate and disease control rate) in a patient population with LAG-3 positive malignant tumors (e.g., gastric tumor or gastroesophageal junction tumor) than in a general patient population having a mix of LAG-3-negative malignant tumors and LAG-3-positive malignant tumors. In order to improve the treatment of malignant tumors, in one aspect, the present invention provides identifying a patient as having a LAG-3-positive tumor and providing an immunotherapy of a LAG-3 inhibitor (e.g., anti-LAG-3 antibody), a PD-1 pathway inhibitor (e.g., anti-PD-1 antibody), and one or more chemotherapeutic agents.

One aspect of the invention relates to a method of inhibiting the growth of a malignant tumor in a human patient, the method comprising administering to the patient an effective amount of each of: (a) a LAG-3 antagonist; (b) a PD-1 pathway inhibitor; and (c) one or more chemotherapeutic agents. Another aspect of the invention relates to a method of treating cancer in a human patient, the method comprising administering to the patient an effective amount of each of: (a) a LAG-3 antagonist; (b) a PD-1 pathway inhibitor; and (c) one or more chemotherapeutic agents. One aspect of the invention relates to a method of treating recurrent, locally advanced or metastatic gastric cancer or gastroesophageal junction adenocarcinoma in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more standard-of-care therapeutic regimens. In one embodiment, the present invention relates to a method of treating gastric cancer or gastroesophageal junction cancer in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more chemotherapeutic agents selected from the group consisting of XELOX, FOLFOX, and SOX. In one embodiment, the invention includes a method of selecting a malignant tumor in a human patient for immunotherapy, comprising: (a) determining the level of LAG-3 expression in a tumor sample; and (b) selecting the tumor for immunotherapy if the tumor is a LAG-3 positive tumor. In one embodiment, the invention includes a method of identifying a malignant tumor in a human patient as eligible for immunotherapy, comprising: (a) determining the level of LAG-3 expression in a tumor sample; and (b) identifying the tumor as eligible for immunotherapy if the tumor is a LAG-3 positive tumor. In one embodiment, the invention includes a method of identifying a malignant tumor in a human patient that is likely to be responsive to a immunotherapy, the method comprising: (a) determining the level of LAG-3 expression in a tumor sample; and (b) identifying the tumor as likely to be responsive to treatment if the tumor is a LAG-3 positive tumor. In one embodiment, the invention includes a method of identifying a malignant tumor in a human patient that is likely to be responsive to a immunotherapy, the method comprising: (a) determining the level of LAG-3 expression in a tumor sample; and (b) identifying the tumor as likely to be responsive to treatment if the tumor is a LAG-3 positive tumor. In one embodiment, the invention includes a method of classifying a malignant tumor in a human patient as likely to be responsive to a immunotherapy, the method comprising: (a) determining the level of LAG-3 expression in a tumor sample; and (b) classifying the tumor as likely to be responsive to immunotherapy if the tumor is a LAG-3 positive tumor. In some embodiments, the immunotherapy comprises contacting the tumor with a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody. In certain embodiments, any of the present methods further comprise determining PD-L1 expression in the tumor sample.

In one embodiment, the invention includes a method of identifying a patient with a malignant tumor who is likely to respond to a immunotherapy, the method comprising: (a) determining the level of LAG-3 expression in a tumor sample; and (b) identifying the patient who is likely to respond to treatment if the tumor is a LAG-3 positive tumor. In one embodiment, the invention includes a method of selecting a patient with a malignant tumor for immunotherapy, the method comprising: (a) determining the level of LAG-3 expression in a tumor sample; and (b) selecting the patient for immunotherapy if the tumor is a LAG-3 positive tumor. In some embodiments, the immunotherapy comprises contacting the tumor with a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody. In certain embodiments, any of the present methods further comprise determining PD-L1 expression in the tumor sample.

In one embodiment, the invention includes a method of treating a malignant tumor in a human patient, comprising: administering to the patient an immunotherapy disclosed herein; wherein the patient is predicted to respond to treatment with the LAG-3 inhibitor, PD-1 pathway inhibitor, and chemotherapeutic agents based upon LAG-3 expression or based upon LAG-3 and PD-L1 expression in a sample of the patient's tumor. In some embodiments, the LAG-3 inhibitor is an anti-LAG-3 antibody and the PD-1 pathway inhibitor is an anti-PD-1 antibody.

In one embodiment, the invention includes a method of treating a tumor in a human patient, comprising: administering to the patient an immunotherapy disclosed herein; wherein the patient is predicted to respond to treatment with the LAG-3 inhibitor, PD-1 pathway inhibitor, and chemotherapeutic agents based upon having a high tumor mutational burden (TMB) status. In one embodiment, the invention includes a method of treating cancer in a human patient, comprising: administering to the patient an immunotherapy disclosed herein; wherein the patient is predicted to respond to treatment with the LAG-3 inhibitor, PD-1 pathway inhibitor, and chemotherapeutic agents based upon having a high tumor mutational burden (TMB) status. In embodiments the cancer is gastric cancer or gastroesophageal junction cancer.

In one embodiment, the invention includes a method of treating a tumor in a human patient, comprising: administering to the patient an immunotherapy disclosed herein; wherein the patient is predicted to respond to treatment with the LAG-3 inhibitor, PD-1 pathway inhibitor, and chemotherapeutic agents based upon having a high degree of microsatellite instability (MSI-H). In one embodiment, the invention includes a method of treating cancer in a human patient, comprising: administering to the patient an immunotherapy disclosed herein; wherein the patient is predicted to respond to treatment with the LAG-3 inhibitor, PD-1 pathway inhibitor, and chemotherapeutic agents based upon having a high degree of microsatellite instability (MSI-H). In embodiments the cancer is gastric cancer or gastroesophageal junction cancer.

In one embodiment, the invention includes a method of treating a malignant tumor in a human patient in need thereof, comprising: (a) determining the level of LAG-3 expression or the level of LAG-3 and PD-L1 expression in a tumor sample; and (b) administering to the patient a therapeutically effective amount of a LAG-3 inhibitor if the tumor is a LAG-3 positive tumor or a LAG-3 positive PD-L1 positive tumor. In one embodiment, the invention includes a method for treating a malignant tumor in a human patient in need thereof, comprising: (a) identifying the patient as having a LAG-3 positive malignant tumor or a LAG-3 positive PD-L1 positive malignant tumor; and (b) administering to the patient a therapeutically effective amount of a LAG-3 inhibitor, a PD-1 pathway inhibitor, and one or more chemotherapeutic agents. In one embodiment, the invention includes a method for treating a malignant tumor in a human patient in need thereof comprising administering to the patient a therapeutically effective amount of a LAG-3 inhibitor, wherein the patient is identified as having a LAG-3 positive malignant tumor or a LAG-3 positive PD-L1 positive malignant tumor prior to the administration. In some embodiments, the LAG-3 inhibitor is an anti-LAG-3 antibody and the PD-1 pathway inhibitor is an anti-PD-1 antibody.

In one embodiment, the invention includes a method of treating a malignant tumor in a human patient in need thereof, comprising: (a) determining the level of LAG-3 expression or the level of LAG-3 and PD-L1 expression in a tumor sample; and (b) administering to the patient a therapeutically effective amount of a LAG-3 inhibitor, a PD-1 pathway inhibitor, and one or more chemotherapeutic agents if the tumor is a LAG-3 positive tumor or a LAG-3 positive/PD-L1 positive tumor. In one embodiment, the invention includes a method for treating a malignant tumor in a human patient in need thereof, comprising: (a) identifying the patient as having a LAG-3 positive malignant tumor or a LAG-3 positive/PD-L1 positive malignant tumor; and (b) administering to the patient a therapeutically effective amount of a LAG-3 inhibitor, a PD-1 pathway inhibitor, and one or more chemotherapeutic agents. In some embodiments, the LAG-3 inhibitor is an anti-LAG-3 antibody, and the PD-1 pathway inhibitor is an anti-PD-1 antibody.

In one embodiment, the invention includes a method of treating a malignant tumor in a human patient in need thereof, comprising: (a) determining the level of LAG-3 expression or the level of LAG-3 and PD-L1 expression in a tumor sample; and (b) administering to the patient a therapeutically effective amount of a LAG-3 inhibitor, a PD-1 pathway inhibitor, and one or more chemotherapeutic agents if the tumor is a LAG-3 positive tumor or a LAG-3 positive PD-L1 positive tumor. In one embodiment, the invention includes a method for treating a malignant tumor in a human patient in need thereof, comprising: (a) identifying the patient as having a LAG-3 positive malignant tumor or a LAG-3 positive PD-L1 positive malignant tumor; and (b) administering to the patient a therapeutically effective amount of a LAG-3 inhibitor, a PD-1 pathway inhibitor, and one or more chemotherapeutic agents. In one embodiment, the invention includes a method for treating a malignant tumor in a human patient in need thereof comprising administering to the patient a therapeutically effective amount of a LAG-3 inhibitor, a PD-1 pathway inhibitor, and one or more chemotherapeutic agents wherein the patient is identified as having a LAG-3 positive malignant tumor or a LAG-3 positive PD-L1 positive malignant tumor prior to the administration. In some embodiments, the LAG-3 inhibitor is an anti-LAG-3 antibody and the PD-1 pathway inhibitor is an anti-PD-1 antibody.

In another embodiment, the invention includes a method for treating a malignant tumor in a human patient in need thereof comprising administering to the patient an immunotherapy disclosed herein, wherein the patient is identified as having a LAG-3 positive malignant tumor or a LAG-3 positive PD-L1 positive malignant tumor prior to the administration. In some embodiments, the immunotherapy comprises administering a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody.

In certain embodiments, the invention includes a method for extending a progression-free survival period for over 12 months in a human patient afflicted with a malignant tumor comprising administering to the patient an immunotherapy disclosed herein, wherein the patient is identified as having a LAG-3 positive malignant tumor or a LAG-3 positive PD-L1 positive malignant tumor prior to the administration and wherein the patient demonstrates progression-free survival for over 12 months. In some embodiments, the progression-free survival of the patient can be extended, after the administration, for over about 13 months, about 14 months, about 15 months, about 16 months, about 17 months, about 18 months, about 2 years, about 3 years, about 4 years, about 5 years, about 6 years, about 7 years, about 8 years, about 9 years, or about 10 years. In some embodiments, the immunotherapy comprises administering a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody.

In still other embodiments, the invention is includes a method for reducing a tumor size at least by 10% in a human patient afflicted with a malignant tumor comprising administering to the patient an immunotherapy disclosed herein, wherein the patient is identified as having a LAG-3 positive malignant tumor (e.g., gastric cancer or gastroesophageal junction cancer) or a LAG-3 positive PD-L1 positive malignant tumor prior to the administration and wherein the administration reduces the tumor size at least about 10%, about 20%, about 30%, about 40%, about 50%, about 60%, about 70%, about 80%, about 90%, or 100% compared to the tumor size prior to the administration. In some embodiments, the method comprises identifying the patient as having a LAG-3 positive malignant tumor or a LAG-3 positive PD-L1 positive malignant tumor prior to the administration. In some embodiments, the LAG-3 inhibitor is an anti-LAG-3 antibody and the PD-1 pathway inhibitor is an anti-PD-1 antibody.

The invention can also include a method of preventing a relapse and/or inducing a remission in a patient comprising administering to the patient an immunotherapy disclosed herein, wherein the patient is identified as having a LAG-3-positive malignant tumor (e.g., gastric cancer or gastroesophageal junction cancer) or a LAG-3 positive PD-L1 positive malignant tumor prior to the administration. In some embodiments, the method of the invention comprises (i) identifying a patient as having a LAG-3-positive malignant tumor or a LAG-3 positive PD-L1 positive malignant tumor; (ii) administering to the patient an immunotherapy disclosed herein. In some embodiments, the immunotherapy comprises administering a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody.

In certain embodiments, the invention includes a method for increasing an objective response rate to be higher than 55% in a patient population, wherein each patient of the patient population is afflicted with a malignant tumor, in a cancer treatment comprising administering to the patient an immunotherapy disclosed herein, wherein each patient is identified as having a LAG-3 positive malignant tumor (e.g., gastric cancer or gastroesophageal junction cancer) or a LAG-3 positive PD-L1 positive malignant tumor prior to the administration and wherein the objective response rate is higher than about 55%, about 60%, about 65%, about 70%, or about 75%. In some embodiments, the method comprises identifying the patient as having a LAG-3 positive malignant tumor or a LAG-3 positive PD-L1 positive malignant tumor prior to the administration. In some embodiments, the immunotherapy comprises administering a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody.

In certain embodiments, the invention includes a method for increasing a disease control rate to be higher than 55% in a patient population, wherein each patient of the patient population is afflicted with a malignant tumor, in a cancer treatment comprising administering to the patient an immunotherapy disclosed herein, wherein each patient is identified as having a LAG-3 positive malignant tumor (e.g., gastric cancer or gastroesophageal junction cancer) or a LAG-3 positive PD-L1 positive malignant tumor prior to the administration and wherein the disease control rate is higher than about 55%, about 60%, about 65%, about 70%, or about 75%. In some embodiments, the method comprises identifying the patient as having a LAG-3 positive malignant tumor or a LAG-3 positive PD-L1 positive malignant tumor prior to the administration. In some embodiments, the immunotherapy comprises administering a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody.

In other embodiments, each patient in the methods experiences (i) extended progression-free survival for over 12 months, (ii) tumor size reduction at least about 10%, about 20%, about 30%, about 40%, or about 50% compared to the tumor size prior to the administration, or (iii) both. In some embodiments, the patient population can be at least 100 patients having a LAG-3 positive malignant tumor (e.g., gastric cancer or gastroesophageal junction cancer) or a LAG-3 positive PD-L1 positive malignant tumor. In some embodiments, the patient population can be at least about 200, 300, 400, 500, 600, 700, 800, 900, or 1000 patients having a LAG-3 positive malignant tumor or a LAG-3 positive PD-L1 positive malignant tumor.

In further embodiments, the invention provides a method for selecting a human patient suitable for a combination therapy comprising: (a) identifying a patient as having a LAG-3 positive malignant tumor or a LAG-3 positive PD-L1 positive malignant tumor; and (b) instructing a healthcare provider to administer to the patient an immunotherapy disclosed herein. The method can further comprise administering an immunotherapy disclosed herein. In some embodiments, the immunotherapy comprises administering a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody. In some embodiments, the administration treats the malignant tumor.

The methods of the invention, as a result of the administration of an immunotherapy disclosed herein, can treat the malignant tumor, reduce the tumor size, inhibit growth of the tumor, eliminate the tumor from the patient, prevent a relapse of a tumor, induce a remission in a patient, or any combination thereof. In certain embodiments, the administration of an immunotherapy disclosed herein induces a complete response. In other embodiments, the administration of the immunotherapy disclosed herein induces a partial response. In some embodiments, the immunotherapy comprises administering a therapeutically effective amount of a LAG-3 inhibitor, a PD-1 pathway inhibitor, and one or more chemotherapeutic agents. In some embodiments, the LAG-3 inhibitor is an anti-LAG-3 antibody and the PD-1 pathway inhibitor is an anti-PD-1 antibody.

In some embodiments, the LAG-3 positive tumor comprises at least about 1%, at least about 2%, at least about 3%, at least about 4%, at least about 5%, at least about 7%, at least about 10%, at least about 15%, at least about 20%, at least about 25%, at least about 30%, at least about 40%, at least about 50%, at least about 60%, at least about 70%, at least about 80%, at least about 90%, or 100% cells expressing LAG-3. In some embodiments, the cells expressing LAG-3 comprise tumor infiltrating lymphocyte.

In some embodiments, the identifying comprises determining LAG-3 expression in the malignant tumor.

In some embodiments, LAG-3 expression is determined by receiving the results of an assay capable of determining LAG-3 expression.

In certain embodiments, any of the present methods further comprise determining PD-L1 expression in the tumor sample.

In certain embodiments, any of the present methods further comprise identifying the patient as having a PD-L1 positive malignant tumor prior to the administration. In certain embodiments, any of the present methods further comprise determining PD-L1 expression in the malignant tumor.

In certain embodiments of any of the present methods, the patient is identified as having a PD-L1 positive malignant tumor prior to the administration. In certain embodiments of any of the present methods, the patient is identified as having a PD-L1 negative malignant tumor prior to the administration.

Method for determining PD-L1 expression in a tumor sample, methods for identifying the patient as having a PD-L1 positive malignant tumor, and methods for determining PD-L1 expression in a malignant tumor have been disclosed in PCT/US2016/029878, the teachings of which are hereby incorporated by reference.

**Measurement of LAG-3 expression**

In certain embodiments, identifying a patient suitable for a LAG-3 inhibitor/ PD-1 pathway inhibitor/chemotherapy combination therapy for the present methods includes measuring or assessing a LAG-3 expression in a malignant tumor test tissue sample comprising tumor cells and tumor infiltrating inflammatory cells. The phrases "tumors expressing LAG-3," "LAG-3 expressing tumor," "LAG-3 positive tumor," and "LAG-3 expression positive tumor" are used interchangeably herein and encompass tumors comprising LAG-3 expressing tumor-infiltrating lymphocytes. The methods of measuring or assessing the LAG-3 expression can be achieved by any methods applicable.

In order to assess LAG-3 expression, in one embodiment, a test tissue sample is obtained from the patient who is in need of the therapy. In some embodiments, a test tissue sample includes, but is not limited to, any clinically relevant tissue sample, such as a tumor biopsy, a core biopsy tissue sample, a fine needle aspirate, or a sample of bodily fluid, such as blood, plasma, serum, lymph, ascites fluid, cystic fluid, or urine. In some embodiments, the test tissue sample is from a primary tumor. In some embodiments, the test tissue sample is from a metastasis. In some embodiments, test tissue samples are taken from a subject at multiple time points, for example, before treatment, during treatment, and/or after treatment. In some embodiments, test tissue samples are taken from different locations in the subject, for example, a sample from a primary tumor and a sample from a metastasis in a distant location.

In some embodiments, the test tissue sample is a paraffin-embedded fixed tissue sample. In some embodiments, the test tissue sample is a formalin-fixed paraffin embedded (FFPE) tissue sample. In some embodiments, the test tissue sample is a fresh tissue (e.g., tumor) sample. In some embodiments, the test tissue sample is a frozen tissue sample. In some embodiments, the test tissue sample is a fresh frozen (FF) tissue (e.g., tumor) sample. In some embodiments, the test tissue sample is a cell isolated from a fluid. In some embodiments, the test tissue sample comprises circulating tumor cells (CTCs). In some embodiments, the test tissue sample comprises tumor-infiltrating lymphocytes (TILs). In some embodiments, the test tissue sample comprises tumor cells and tumor-infiltrating lymphocytes (TILs). In some embodiments, the test tissue sample comprises circulating lymphocytes. In some embodiments, the test tissue sample is an archival tissue sample. In some embodiments, the test tissue sample is an archival tissue sample with known diagnosis, treatment, and/or outcome history. In some embodiments, the sample is a block of tissue. In some embodiments, the test tissue sample is dispersed cells. In some embodiments, the sample size is from about 1 cell to about $1 \times 10^6$ cells or more. In some embodiments, the sample size is about 1 cell to about $1 \times 10^5$ cells. In some embodiments, the sample size is about 1 cell to about 10,000 cells. In some embodiments, the sample size is about 1 cell to about 1,000 cells. In some embodiments, the sample size is about 1 cells to about 100 cells. In some embodiments, the sample size is about 1 cell to about 10 cells. In some embodiments, the sample size is a single cell.

In another embodiment, the assessment of LAG-3 expression can be achieved without obtaining a test tissue sample. In some embodiments, selecting a suitable patient includes (i) optionally providing a test tissue sample obtained from a patient with cancer of the tissue, the test tissue sample comprising tumor cells and/or tumor-infiltrating inflammatory cells; and (ii) assessing the proportion of cells in the test tissue sample that express LAG-3 on the surface of the cells based on an assessment that the proportion of cells in the test tissue sample that express LAG-3 on the cell surface is higher than a predetermined threshold level.

In any of the methods comprising the measurement of LAG-3 expression in a test tissue sample, however, it should be understood that the step comprising the provision of a test tissue sample obtained from a patient is an optional step. That is, in certain embodiments the method includes this step, and in other embodiments, this step is not included in the method. It should also be understood that in certain embodiments the "measuring" or "assessing" step to identify, or determine the number or proportion of, cells in the test tissue sample that express LAG-3 is performed by a transformative method of assaying for LAG-3 expression, for example by performing a reverse transcriptase-polymerase chain reaction (RT-PCR) assay or an IHC assay. In certain other embodiments, no transformative step is involved and LAG-3 expression is assessed by, for example, reviewing a report of test results from a laboratory. In some embodiments, LAG-3 expression is assessed by reviewing the results of an immunohistochemistry assay from a laboratory. In certain embodiments, the steps of the methods up to, and including, assessing LAG-3 expression provides an intermediate result that may be provided to a physician or other healthcare provider for use in selecting a suitable candidate for the combination therapy of a LAG-3 inhibitor, a PD-1 pathway inhibitor, and one or more chemotherapeutic agents. In certain embodiments, the steps that provide the intermediate result is performed by a medical practitioner or someone acting under the direction of a medical practitioner. In other embodiments, these steps are performed by an independent laboratory or by an independent person such as a laboratory technician.

In certain embodiments of any of the present methods, the proportion of cells that express LAG-3 is assessed by performing an assay to detect the presence of LAG-3 RNA. In further embodiments, the presence of LAG-3 RNA is detected by RT-PCR, *in situ* hybridization or RNase protection. In some embodiments, the presence of LAG-3 RNA is detected by an RT-PCR based assay. In some embodiments, scoring the RT-PCR based assay comprises assessing the level of LAG-3 RNA expression in the test tissue sample relative to a predetermined level.

In other embodiments, the proportion of cells that express LAG-3 is assessed by performing an assay to detect the presence of LAG-3 polypeptide. In further embodiments, the presence of LAG-3 polypeptide is detected by IHC, enzyme-linked immunosorbent assay (ELISA), *in vivo* imaging, or flow cytometry. In some embodiments, LAG-3 expression is assayed by IHC. In other embodiments of all of these methods, cell surface expression of LAG-3 is assayed using, *e.g.,* IHC or *in vivo* imaging.

In other embodiments, the proportion of cells that express LAG-3 in the test tissue sample is assessed by flow cytometry. In some embodiments, the test tissue sample assayed by flow cytometry comprises tumor infiltrating immune cells. In some embodiments, the malignant tumor is a hematological malignancy and the tissue sample assayed by flow cytometry comprises peripheral blood cells. In some embodiments, the flow cytometry is a multiplex assay. In some embodiments, scoring the flow cytometry comprises detecting the expression of markers comprising LAG-3, CD4, CD8, FOXP3, and any combination thereof. In some embodiments, scoring the flow cytometry comprises assessing the proportion of T cells in the test tissue sample that express LAG-3. In some embodiments, scoring the flow cytometry comprises assessing the proportion of CD8+ T cells in the test tissue sample that express LAG-3. In some embodiments, scoring the flow cytometry comprises assessing the proportion of CD4+ T cells in the test tissue sample that express LAG-3. In some embodiments, scoring the flow cytometry comprises assessing the proportion of FOXP3+ T cells in the test tissue sample that express LAG-3.

In certain embodiments of any of the present methods, the proportion of cells that express LAG-3 in the test tissue sample is assessed by performing an assay to detect the presence of LAG-3 polypeptide. In some embodiments, the presence of LAG-3 polypeptide is detected by an immunohistochemistry assay. In some embodiments, the test tissue sample is a tumor biopsy. In some embodiments, the test tissue sample is a formalin-fixed paraffin embedded (FFPE) sample.

In some embodiments, the immunohistochemistry assay is a monoplex assay. In some embodiments, the immunohistochemistry assay is a multiplex assay. In some embodiments, the multiplex immunohistochemistry assay is capable of detecting the presence of CD4, CD8, FOXP3, or any combination thereof.

In some embodiments, the immunohistochemistry assay comprises contacting the tumor sample with the 17B4 mouse anti-human LAG-3 IgG1 monoclonal antibody. In some embodiments, the immunohistochemistry assay comprises contacting the tumor sample with an anti-LAG-3 antibody comprising heavy and light chain variable regions comprising the sequences set forth in SEQ ID NOs: 3 and 5, respectively. In some embodiments, the immunohistochemistry assay comprises contacting the tumor sample with the SP346 rabbit anti-human LAG-3 IgG monoclonal antibody. In some embodiments, the immunohistochemistry assay comprises contacting the tumor sample with the 11E3 (Novusbio), 874501 (Novusbio), or EPR4392(2) (Abcam) anti-human LAG-3 monoclonal antibody.

In some embodiments, the immunohistochemistry assay is scored at a low magnification. In some embodiments, low magnification is about 20X. In some embodiments, the immunohistochemistry assay is scored at high magnification. In some embodiments, high magnification is about 40X.

In some embodiments, the immunohistochemistry assay is scored by an image analysis software. In some embodiments, the immunohistochemistry assay is scored by pathologist visual immune score. In some embodiments, the immunohistochemistry assay is scored manually.

In some embodiments, scoring the immunohistochemistry assay comprises assessing the proportion of cells in the test tissue sample that express LAG-3. In some embodiments, scoring the immunohistochemistry assay comprises assessing the proportion of immune cells in the test tissue sample that express LAG-3. In some embodiments, scoring the immunohistochemistry assay comprises assessing the proportion of T cells in the test tissue sample that express LAG-3. In some embodiments, scoring the immunohistochemistry assay comprises assessing the proportion of CD8+ T cells in the test tissue sample that express LAG-3. In some embodiments, scoring the immunohistochemistry assay comprises assessing the proportion of CD4+ T cells in the test tissue sample that express LAG-3. In some embodiments, scoring the immunohistochemistry assay comprises assessing the proportion of FOXP3+ T cells in the test tissue sample that express LAG-3.

LAG-3 polypeptide localization includes partial membrane/cytoplasmic localization, dot like localization, and complete membrane/cytoplasmic localization. In some embodiments, cells with partial membrane/cytoplasmic LAG-3 localization are scored. In some embodiments, cells with dot-like LAG-3 localization are scored. In some embodiments, cells with complete membrane/cytoplasmic LAG-3 localization are scored. In some embodiments, cells with any LAG-3 localization pattern are scored.

In some embodiments, the immunohistochemistry assay is a multiplex assay that further comprises detecting the expression of MHC Class II by the tumor cells. In some embodiments, scoring the immunohistochemistry assay comprises assessing the proportion of cells in the test tissue sample that expresses MHC Class II. In some embodiments, scoring the immunohistochemistry assay comprises assessing the proportion of non-immune cells in the test tissue sample that expresses MHC Class II.

Imaging techniques have provided important tools in cancer research and treatment. Recent developments in molecular imaging systems, including positron emission tomography (PET), single-photon emission computed tomography (SPECT), fluorescence reflectance imaging (FRI), fluorescence-mediated tomography (FMT), bioluminescence imaging (BLI), laser-scanning confocal microscopy (LSCM) and multiphoton microscopy (MPM), will likely herald even greater use of these techniques in cancer research. Some of these molecular imaging systems allow clinicians to not only see where a tumor is located in the body, but also to visualize the expression and activity of specific molecules, cells, and biological processes that influence tumor behavior and/or responsiveness to therapeutic drugs (Condeelis and Weissleder, Cold Spring Harb. Perspect. Biol. 2(12):a003848 (2010)). Antibody specificity, coupled with the sensitivity and resolution of PET, makes immunoPET imaging particularly attractive for monitoring and assaying expression of antigens in tissue samples (McCabe and Wu, Cancer Biother. Radiopharm. 25(3):253-61 (2010); Olafsen et al., Protein Eng. Des. Sel. 23(4):243-9 (2010)). In certain embodiments of any of the present methods, LAG-3 expression is assayed by immunoPET imaging. In certain embodiments of any of the present methods, the proportion of cells in a test tissue sample that express LAG-3 is assessed by performing an assay to determine the presence of LAG-3 polypeptide on the surface of cells in the test tissue sample. In certain embodiments, the test tissue sample is a FFPE tissue sample. In other embodiments, the presence of LAG-3 polypeptide is determined by IHC assay. In further embodiments, the IHC assay is performed using an automated process. In some embodiments, the IHC assay is performed using an anti-LAG-3 mAb to bind to the LAG-3 polypeptide.

**Assaying LAG-3 Expression by Automated IHC**

In one embodiment of the present methods, an automated IHC method is used to assay the expression of LAG-3 in FFPE tissue specimens. This disclosure provides methods for detecting the presence of human LAG-3 antigen in a test tissue sample, or quantifying the level of human LAG-3 antigen or the proportion of cells in the sample that express the antigen, which methods comprise contacting the test sample, and a negative control sample, with a mAb that specifically binds to human LAG-3, under conditions that allow for formation of a complex between the antibody or portion thereof and human LAG-3. In certain embodiments, the test and control tissue samples are FFPE samples. The formation of a complex is then detected, wherein a difference in complex formation between the test sample and the negative control sample is indicative of the presence of human LAG-3 antigen in the sample. Various methods are used to quantify LAG-3 expression.

In a particular embodiment, the automated IHC method comprises: (a) deparaffinizing and rehydrating mounted tissue sections in an autostainer; (b) retrieving antigen in an autostainer; (c) setting up reagents on an autostainer; and (d) running the autostainer to include steps of neutralizing endogenous peroxidase in the tissue specimen; blocking non-specific protein-binding sites on the slides; incubating the slides with primary Ab; incubating with a postprimary blocking agent; incubating with a postprimary antibody detection agent, such as another antibody that may or may not be conjugated to a detection enzyme; incubating with a polymeric-enzyme detection reagent; adding a chromogen substrate and developing; and counterstaining with hematoxylin. In some embodiments, the retrieving antigen comprises using any heat based antigen retrieval device.

In some embodiments, for assessing LAG-3 expression in tumor tissue samples, a pathologist examines the number of LAG-3+ tumor cells in each field under a microscope and mentally estimates the percentage of cells that are positive, then averages them to come to the final percentage. The different staining intensities are defined as 0/negative, 1+/weak, 2+/moderate, and 3+/strong. Typically, percentage values are first assigned to the 0 and 3+ buckets, and then the intermediate 1+ and 2+ intensities are considered. For highly heterogeneous tissues, the specimen is divided into zones, and each zone is scored separately and then combined into a single set of percentage values. The percentages of negative and positive cells for the different staining intensities are determined from each area and a median value is given to each zone. A final percentage value is given to the tissue for each staining intensity category: negative, 1+, 2+, and 3+. The sum of all staining intensities needs to be 100%.

In some embodiments, staining is also assessed in tumor-infiltrating inflammatory cells such as macrophages and lymphocytes. Macrophages and lymphocytes are assessed for LAG-3 staining and only recorded for all samples as being positive or negative for each cell category. Staining is also characterized according to an outside/inside tumor immune cell designation. "Inside" means the immune cell is within the tumor tissue and/or on the boundaries of the tumor region without being physically intercalated among the tumor cells. "Outside" means that there is no physical association with the tumor, the immune cells being found in the periphery associated with connective or any associated adjacent tissue.

In certain embodiments of these scoring methods, the samples are scored by two or more pathologists operating independently, and the scores are subsequently consolidated. In certain other embodiments, the identification of positive and negative cells is scored using appropriate software.

A histoscore (H-score) is used as a more quantitative measure of the IHC data. The histoscore is calculated as follows:

Histoscore = [(% tumor x 1 (low intensity)) + (% tumor x 2 (medium intensity)) + (% tumor x 3 (high intensity)]

To determine the histoscore, the pathologist estimates the percentage of stained cells in each intensity category within a specimen. Because expression of most biomarkers is heterogeneous the histoscore is a truer representation of the overall expression. The final histoscore range is 0 (minimum score, no expression) to 300 (maximum score, strong and inclusive expression).

### 3. LAG-3 inhibitors

In one aspect, the invention features methods of using a LAG-3 inhibitor in the treatment of malignant tumors. As used herein LAG-3 inhibitor includes, but is not limited to, LAG-3 binding agents and soluble LAG-3 polypeptides. LAG-3 binding agents include antibodies that specifically bind to LAG-3.

In some embodiments, a LAG-3 inhibitor is a LAG-3-binding agent, for example an anti-LAG-3 antibody. In some embodiments, the LAG-3 inhibitor is a soluble LAG-3 polypeptide, for example, a LAG-3-Fc fusion polypeptide capable of binding to MHC Class II.

Anti-human-LAG-3 antibodies (or VH/VL domains derived therefrom) suitable for use in the invention can be generated using methods well known in the art. Alternatively, art recognized anti-LAG-3 antibodies can be used.

In some embodiments, the anti-LAG-3 antibody is BMS-986016 comprising heavy and light chains comprising the sequences shown in SEQ ID NOs:1 and 2, respectively, or antigen binding fragments and variants thereof, as described in PCT/US13/48999, the teachings of which are hereby incorporated by reference. In some embodiments, the BMS-986016 antibody does not comprise the heavy chain terminal lysine amino acid of SEQ ID NO:1.

In other embodiments, the antibody has the heavy and light chain CDRs or variable regions of BMS-986016. Accordingly, in one embodiment, the antibody comprises CDR1, CDR2, and CDR3 domains of the VH region of BMS-986016 having the sequence set forth in SEQ ID NO:3, and CDR1, CDR2 and CDR3 domains of the VL region of BMS-986016 having the sequence set forth in SEQ ID NO:5. In another embodiment, the antibody comprises CDR1, CDR2 and CDR3 domains comprising the sequences set forth in SEQ ID NOs:7, 8, and 9, respectively, and CDR1, CDR2 and CDR3 domains comprising the sequences set forth in SEQ ID NOs:10, 11, and 12, respectively. In another embodiment, the antibody comprises VH and/or VL regions comprising the amino acid sequences set forth in SEQ ID NO:3 and/or SEQ ID NO: 5, respectively. In another embodiment, the antibody comprises heavy chain variable (VH) and/or light chain variable (VL) regions encoded by the nucleic acid sequences set forth in SEQ ID NO:4 and/or SEQ ID NO:6, respectively. In another embodiment, the antibody competes for binding with and/or binds to the same epitope on LAG-3 as the above-mentioned antibodies. In another embodiment, the antibody binds an epitope of human LAG-3 comprising the amino acid sequence PGHPLAPG (SEQ ID NO: 14). In another embodiment, the antibody binds an epitope of human LAG-3 comprising the amino acid sequence HPAAPSSW (SEQ ID NO:15) or PAAPSSWG (SEQ ID NO:16).

In another embodiment, the antibody has at least about 90% variable region amino acid sequence identity with the above-mentioned antibodies (e.g., at least about 90%, 95% or 99% variable region identity with SEQ ID NO:3 or SEQ ID NO:5).

In some embodiments, the anti-LAG-3 antibody or antigen-binding portion thereof cross-competes with BMS-986016 (relatlimab) for binding to human LAG-3. In other embodiments, the anti- LAG-3 antibody or antigen-binding portion thereof binds to the same epitope as BMS-986016 (relatlimab). In some embodiments, the anti-LAG-3 antibody or antigen-binding portion thereof cross-competes with TSR-033 for binding to human LAG-3. In other embodiments, the anti-LAG-3 antibody or antigen-binding portion thereof binds to the same epitope as TSR-033. In some embodiments, the anti-LAG-3 antibody or antigen-binding portion thereof cross-competes with TSR-075 for binding to human LAG-3. In other embodiments, the anti-LAG-3 antibody or antigen-binding portion thereof binds to the same epitope as TSR-075. In some embodiments, the anti- LAG-3 antibody is a chimeric antibody, a humanized antibody, a human monoclonal antibody, or an antigen-binding portion thereof. In other embodiments, the anti- LAG-3 antibody or antigen-binding portion thereof comprises a heavy chain constant region of a human IgGl isotype or a human IgG4 isotype. In particular embodiments, the anti-PD-1 antibody or antigen-binding portion thereof is BMS-986016 (relatlimab). In some embodiments, the anti- LAG-3 antibody or antigen-binding portion thereof is a biosimilar of BMS-986016 (relatlimab). In particular embodiments, the anti-PD-1 antibody or antigen-binding portion thereof is TSR-033. In some embodiments, the anti- LAG-3 antibody or antigen-binding portion thereof is a biosimilar of TSR-033. In particular embodiments, the anti-PD-1 antibody or antigen-binding portion thereof is TSR-075. In some embodiments, the anti- LAG-3 antibody or antigen-binding portion thereof is a biosimilar of TSR-075.

In some embodiments, art recognized anti-LAG-3 antibodies can be used in the therapeutic methods of the invention. For example, the anti-human LAG-3 antibody described in US2011/0150892 A1, which is herein incorporated by reference, and referred to as monoclonal antibody 25F7 (also known as "25F7" and "LAG-3.1) can be used. Other art recognized anti-LAG-3 antibodies that can be used include IMP731 (H5L7BW) described in US 2011/007023, MK-4280 (28G-10) described in WO2016028672, REGN3767 described in Journal for ImmunoTherapy of Cancer, (2016) Vol. 4, Supp. Supplement 1 Abstract Number: P195, BAP050 described in WO2017/019894, IMP-701 (LAG-525), aLAG3(0414), aLAG3(0416), Sym022, TSR-033, TSR-075, XmAb22841, MGD013, BI754111, FS118, P 13B02-30, AVA-017 and GSK2831781. These and other anti-LAG-3 antibodies useful in the claimed invention can be found in, for example: US 10,188,730, WO2016/028672, WO2017/106129, WO2017/062888, WO2009/044273, WO2018/069500, WO2016/126858, WO2014/179664, WO2016/200782, WO2015/200119, WO2017/019846, WO2017/198741, WO2017/220555, WO2017/220569, WO2018/071500, WO2017/015560, WO2017/025498, WO2017/087589, WO2017/087901, WO2018/083087, WO2017/149143, WO2017/219995, US2017/0260271, WO2017/086367, WO2017/086419, WO2018/034227, WO18/185046, WO18/185043, WO2018/217940, WO19/011306, WO2018/208868, and WO2014/140180. In one embodiment, the LAG-3 inhibitor is IMP321 (eftilagimod alpha). The contents of each of these references are incorporated by reference herein in their entirety.

Antibodies that compete with any of the above-referenced art-recognized antibodies for binding to LAG-3 also can be used.

In certain embodiments, an anti-LAG-3 antibody is used to determine LAG-3 expression. In some embodiments, an anti-LAG-3 antibody is selected for its ability to bind to LAG-3 in formalin-fixed, paraffin-embedded (FFPE) tissue specimens. In other embodiments, an anti-LAG-3 antibody is capable of binding to LAG-3 in frozen tissues. In further embodiments, an anti-LAG-3 antibody is capable of distinguishing membrane bound, cytoplasmic, and/or soluble forms of LAG-3.

In some embodiments, an anti-LAG-3 antibody useful for assaying, detecting, and/or quantifying LAG-3 expression in accordance with the methods described herein is the 17B4 mouse IgG1 anti-human LAG-3 monoclonal antibody, or an antigen binding fragment thereof. *See, e.g.,* J. Matsuzaki, et al.; PNAS 107, 7875 (2010).

## 4. PD-1 pathway inhibitors

In one aspect, the invention features methods of using a PD-1 inhibitor in the treatment of malignant tumors. As used herein "PD-1 pathway inhibitor" includes, but is not limited to, PD-1 binding agents, PD-L1 binding agent and PD-L2 binding agents. PD-1 binding agents include antibodies that specifically bind to PD-1. PD-L1 and PD-L2 binding agents include antibodies that specifically bind to PD-L1 and/or PD-L2, as well as soluble PD-1 polypeptides that bind to PD-L1 and/or PD-L2.

In some embodiments, PD-1 pathway inhibitor is a PD-1-binding agent, for example an anti-PD-1 antibody. In some embodiments, the PD-1 pathway inhibitor is a PD-L1-binding agent, for example, an anti-PD-L1 antibody. In some embodiments, the PD-1 pathway inhibitor is a PD-L2-binding agent, for example an anti-PD-L2 antibody. In further embodiments, the PD-L1-binding agent is a soluble PD-1 polypeptide, for example, a PD-1-Fc fusion polypeptide capable of binding to PD-L1. In further embodiments, the PD-L2-binding agent is a soluble PD-1 polypeptide, for example, a PD-1-Fc fusion polypeptide capable of binding to PD-L2.

Anti-human-PD-1 antibodies (or VH and/or VL domains derived therefrom) suitable for use in the invention can be generated using methods well known in the art. Alternatively, art recognized anti-PD-1 antibodies can be used. For example, monoclonal antibodies 5C4 (referred to herein as Nivolumab or BMS-936558), 17D8, 2D3, 4H1, 4A11, 7D3, and 5F4, described in WO 2006/121168, incorporated herein by reference in its entirety, can be used. Other known PD-1 antibodies include lambrolizumab (MK-3475) described in WO 2008/156712, and AMP-514 described in WO 2012/145493, which are herein incorporated by reference. Further known PD-1 antibodies and other PD-1 inhibitors include those described in, for example, WO 2009/014708, WO 03/099196, WO 2009/114335 and WO 2011/161699, which are herein incorporated by reference. In one embodiment, the anti-PD-1 antibody is REGN2810. In one embodiment, the anti-PD-1 antibody is PDR001. Another known anti-PD-1 antibody is pidilizumab (CT-011).

In one embodiment, the anti-PD-1 antibody is nivolumab. Nivolumab (also known as "OPDIVO®"; formerly designated 5C4, BMS-936558, MDX-1106, or ONO-4538) is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Patent No. 8,008,449; Wang et al., Cancer Immunol Res. 2(9):846-56 (2014)). In another embodiment, the anti-PD-1 antibody or fragment thereof cross-competes with nivolumab. In other embodiments, the anti-PD-1 antibody or fragment thereof binds to the same epitope as nivolumab. In certain embodiments, the anti-PD-1 antibody has the same CDRs as nivolumab.

In some embodiments, the anti-PD-1 antibody or antigen-binding portion thereof cross-competes with nivolumab for binding to human PD-1. In other embodiments, the anti-PD-1 antibody or antigen-binding portion thereof binds to the same epitope as nivolumab. In some embodiments, the anti-PD-1 antibody is a chimeric antibody, a humanized antibody, a human monoclonal antibody, or an antigen-binding portion thereof. In other embodiments, the anti-PD-1 antibody or antigen-binding portion thereof comprises a heavy chain constant region of a human IgGl isotype or a human IgG4 isotype. In particular embodiments, the anti-PD-1 antibody or antigen-binding portion thereof is nivolumab or pembrolizumab. In some embodiments, the anti-PD-1 antibody or antigen-binding portion thereof is a biosimilar of nivolumab. In some embodiments, the anti-PD-1 antibody or antigen-binding portion thereof is a biosimilar of pembrolizumab.

In some embodiments, the anti-PD-1 antibody comprises heavy and light chains comprising the sequences shown in SEQ ID NOs: 17 and 18, respectively, or antigen binding fragments and variants thereof.

In other embodiments, the antibody has heavy and light chain CDRs or variable regions of nivolumab. Accordingly, in one embodiment, the antibody comprises CDR1, CDR2, and CDR3 domains of the VH of nivolumab having the sequence set forth in SEQ ID NO:19, and CDR1, CDR2 and CDR3 domains of the VL of nivolumab having the sequence set forth in SEQ ID NO:21. In another embodiment, the antibody comprises CDR1, CDR2 and CDR3 domains comprising the sequences set forth in SEQ ID NOs:23, 24, and 25, respectively, and CDR1, CDR2 and CDR3 domains comprising the sequences set forth in SEQ ID NOs:26, 27, and 28, respectively. In another embodiment, the antibody comprises VH and/or VL regions comprising the amino acid sequences set forth in SEQ ID NO: 19 and/or SEQ ID NO: 21, respectively. In another embodiment, the antibody comprises heavy chain variable (VH) and/or light chain variable (VL) regions encoded by the nucleic acid sequences set forth in SEQ ID NO:20 and/or SEQ ID NO:22, respectively. In another embodiment, the antibody competes for binding with and/or binds to the same epitope on PD-1 as the above-mentioned antibodies. In another embodiment, the antibody has at least about 90% variable region amino acid sequence identity with the above-mentioned antibodies (e.g., at least about 90%, 95% or 99% variable region identity with SEQ ID NO: 19 or SEQ ID NO:21).

Human monoclonal antibodies (HuMAbs) that bind specifically to PD-1 with high affinity have been disclosed in U.S. Patent Nos. 8,008,449 and 8,779,105, which are herein incorporated by reference. Other anti-PD-1 mAbs have been described in, for example, U.S. Patent Nos. 6,808,710, 7,488,802, 8,168,757 and 8,354,509, and PCT Publication No. WO 2012/145493, which are herein incorporated by reference. In some embodiments, the anti-PD-1 antibody has been demonstrated to exhibit one or more of the following characteristics: (a) binds to human PD-1 with a $K_D$ of $1 \times 10^{-7}$ M or less, as determined by surface plasmon resonance using a Biacore biosensor system; (b) does not substantially bind to human CD28, CTLA-4 or ICOS; (c) increases T-cell proliferation in a Mixed Lymphocyte Reaction (MLR) assay; (d) increases interferon-γ production in an MLR assay; (e) increases IL-2 secretion in an MLR assay; (f) binds to human PD-1 and cynomolgus monkey PD-1; (g) inhibits the binding of PD-L1 and/or PD-L2 to PD-1; (h) stimulates antigen-specific memory responses; (i) stimulates antibody responses; and (j) inhibits tumor cell growth *in vivo.* Anti-PD-1 antibodies useful for the present invention include mAbs that bind specifically to human PD-1 and exhibit at least one, at least two, at least three, at least four, or at least five of the preceding characteristics. Anti-PD-1 antibodies that exhibit one or more of these characteristics have been disclosed in U.S. Patent Nos. 8,008,449, 8,779,105, 6,808,710, 7,488,802, 8,168,757 and 8,354,509, and PCT Publication No. WO 2012/145493, which are herein incorporated by reference. In another embodiment, the anti-PD-1 antibody is pembrolizumab. Pembrolizumab is a humanized monoclonal IgG4 (S228P) antibody directed against human cell surface receptor PD-1 (programmed death-1 or programmed cell death-1). Pembrolizumab is described, for example, in U.S. Patent Nos. 8,354,509 and 8,900,587, which are herein incorporated by reference.

In some embodiments, the anti-PD-1 antibody or fragment thereof cross-competes with pembrolizumab. In some embodiments, the anti-PD-1 antibody or fragment thereof binds to the same epitope as pembrolizumab. In certain embodiments, the anti-PD-1 antibody has the same CDRs as pembrolizumab. In another embodiment, the anti-PD-1 antibody is pembrolizumab. Pembrolizumab (also known as "KEYTRUDA®", lambrolizumab, and MK-3475) is a humanized monoclonal IgG4 antibody directed against human cell surface receptor PD-1 (programmed death-1 or programmed cell death-1). Pembrolizumab is described, for example, in U.S. Patent Nos. 8,354,509 and 8,900,587, which are herein incorporated by reference; see also http://www.cancer.gov/drugdictionary?cdrid=695789 (last accessed: December 14, 2014).

In other embodiments, the anti-PD-1 antibody or fragment thereof cross-competes with MEDI0608. In still other embodiments, the anti-PD-1 antibody or fragment thereof binds to the same epitope as MEDI0608. In certain embodiments, the anti-PD-1 antibody has the same CDRs as MEDI0608. In other embodiments, the anti-PD-1 antibody is MEDI0608 (formerly AMP-514), which is a monoclonal antibody. MEDI0608 is described, for example, in US Pat. No. 8,609,089B2, which is herein incorporated by reference, or in http://www.cancer.gov/drugdictionary?cdrid=756047 (last accessed December 14, 2014).

In certain embodiments, the first antibody is an anti-PD-1 antagonist. One example of the anti-PD-1 antagonist is AMP-224, which is a B7-DC Fc fusion protein. AMP-224 is discussed in U.S. Publ. No. 2013/0017199, which is herein incorporated by reference, or in http://www.cancer.gov/publications/dictionaries/cancer-drug?cdrid=700595 (last accessed July 8, 2015).

In other embodiments, the anti-PD-1 antibody or fragment thereof cross-competes with BGB-A317. In some embodiments, the anti-PD-1 antibody or fragment thereof binds the same epitope as BGB-A317. In certain embodiments, the anti-PD-1 antibody has the same CDRs as BGB-A317. In certain embodiments, the anti-PD-1 antibody is BGB-A317, which is a humanized monoclonal antibody. BGB-A317 is described in U.S. Publ. No. 2015/0079109, which is herein incorporated by reference.

In some embodiments, the antibody is pidilizumab (CT-011), which is an antibody previously reported to bind to PD-1 but which is believed to bind to a different target. pidilizumab is described in US Pat. No. 8,686,119 B2 or WO 2013/014668 A1, which are herein incorporated by reference.

In certain embodiments, the antibodies that cross-compete for binding to human PD-1 with, or bind to the same epitope region of human PD-1 as, nivolumab are mAbs. For administration to human subjects, these cross-competing antibodies can be chimeric antibodies, or humanized or human antibodies. Such chimeric, humanized or human mAbs can be prepared and isolated by methods well known in the art.

Other anti-PD-1 monoclonal antibodies have been described in, for example, U.S. Patent Nos. 6,808,710, 7,488,802, 8,168,757 and 8,354,509, US Publication No. 2016/0272708, and PCT Publication Nos. WO 2012/145493, WO 2008/156712, WO 2015/112900, WO 2012/145493, WO 2015/112800, WO 2014/206107, WO 2015/35606, WO 2015/085847, WO 2014/179664, WO 2017/020291, WO 2017/020858, WO 2016/197367, WO 2017/024515, WO 2017/025051, WO 2017/123557, WO 2016/106159, WO 2014/194302, WO 2017/040790, WO 2017/133540, WO 2017/132827, WO 2017/024465, WO 2017/025016, WO 2017/106061, WO 2017/19846, WO 2017/024465, WO 2017/025016, WO 2017/132825, and WO 2017/133540 each of which is incorporated by reference in its entirety.

In some embodiments, the anti-PD-1 antibody is selected from the group consisting of nivolumab (also known as OPDIVO®, 5C4, BMS-936558, MDX-1106, and ONO-4538), pembrolizumab (Merck; also known as KEYTRUDA®, lambrolizumab, and MK-3475; *see* WO2008/156712), PDR001 (Novartis; *see* WO 2015/112900), MEDI-0680 (Astra-Zeneca; also known as AMP-514; *see* WO 2012/145493), cemiplimab (Regeneron; also known as REGN-2810; *see* WO 2015/112800), JS001 (TAIZHOU JUNSHI PHARMA; *see* Si-Yang Liu et al., J. Hematol. Oncol. 10:136 (2017)), BGB-A317 (Beigene; *see* WO 2015/35606 and US 2015/0079109), INCSHR1210 (Jiangsu Hengrui Medicine; also known as SHR-1210; *see* WO 2015/085847; Si-Yang Liu et al., J. Hematol. Oncol. 10:136 (2017)), TSR-042 (Tesaro Biopharmaceutical; also known as ANB011; *see* WO2014/179664), GLS-010 (Wuxi/Harbin Gloria Pharmaceuticals; also known as WBP3055; *see* Si-Yang Liu et al., J. Hematol. Oncol. 10:136 (2017)), AM-0001 (Armo), STI-1110 (Sorrento Therapeutics; *see* WO 2014/194302), AGEN2034 (Agenus; *see* WO 2017/040790), MGA012 (Macrogenics, *see* WO 2017/19846), and IBI308 (Innovent; *see* WO 2017/024465, WO 2017/025016, WO 2017/132825, and WO 2017/133540). The contents of each of these references are incorporated by reference herein in their entirety.

Anti-PD-1 antibodies useful for the compositions of the disclosed invention also include antigen-binding portions of the above antibodies. It has been amply demonstrated that the antigen-binding function of an antibody can be performed by fragments of a full-length antibody. Examples of binding fragments encompassed within the term "antigen-binding portion" of an antibody include (i) a Fab fragment, a monovalent fragment consisting of the $V_L$, $V_H$, $C_L$ and $C_{H1}$ domains; (ii) a F(ab')$_2$ fragment, a bivalent fragment comprising two Fab fragments linked by a disulfide bridge at the hinge region; (iii) a Fd fragment consisting of the $V_H$ and $C_{H1}$ domains; and (iv) a Fv fragment consisting of the $V_L$ and $V_H$ domains of a single arm of an antibody.

Anti-PD-1 antibodies usable in the disclosed methods also include isolated antibodies that bind specifically to human PD-1 and cross-compete for binding to human PD-1 with any anti-PD-1 antibody disclosed herein, e.g., nivolumab (*see, e.g.*, U.S. Patent No. 8,008,449 and 8,779,105; WO 2013/173223, which are herein incorporated by reference). In some embodiments, the anti-PD-1 antibody binds the same epitope as any of the anti-PD-1 antibodies described herein, e.g., nivolumab. The ability of antibodies to cross-compete for binding to an antigen indicates that these monoclonal antibodies bind to the same epitope region of the antigen and sterically hinder the binding of other cross-competing antibodies to that particular epitope region. These cross-competing antibodies are expected to have functional properties very similar those of the reference antibody, e.g., nivolumab, by virtue of their binding to the same epitope region of PD-1. Cross-competing antibodies can be readily identified based on their ability to cross-compete with nivolumab in standard PD-1 binding assays such as Biacore analysis, ELISA assays or flow cytometry (*see, e.g.*, WO 2013/173223, which is herein incorporated by reference).

Anti-PD-1 antibodies suitable for use in the disclosed methods are antibodies that bind to PD-1 with high specificity and affinity, block the binding of PD-L1 and or PD-L2, and inhibit the immunosuppressive effect of the PD-1 signaling pathway. In any of the compositions or methods disclosed herein, an anti-PD-1 "antibody" includes an antigen-binding portion or fragment that binds to the PD-1 receptor and exhibits the functional properties similar to those of whole antibodies in inhibiting ligand binding and upregulating the immune system. In certain embodiments, the anti-PD-1 antibody or antigen-binding portion thereof cross-competes with nivolumab for binding to human PD-1. In other embodiments, the anti-PD-1 antibody or antigen-binding portion thereof is a chimeric, humanized or human monoclonal antibody or a portion thereof. In certain embodiments, the antibody is a humanized antibody. In other embodiments, the antibody is a human antibody. Antibodies of an IgG1, IgG2, IgG3 or IgG4 isotype can be used.

In certain embodiments, the anti-PD-1 antibody or antigen-binding portion thereof comprises a heavy chain constant region which is of a human IgG1 or IgG4 isotype. In certain other embodiments, the sequence of the IgG4 heavy chain constant region of the anti-PD-1 antibody or antigen-binding portion thereof contains an S228P mutation which replaces a serine residue in the hinge region with the proline residue normally found at the corresponding position in IgG1 isotype antibodies. This mutation, which is present in nivolumab, prevents Fab arm exchange with endogenous IgG4 antibodies, while retaining the low affinity for activating Fc receptors associated with wild-type IgG4 antibodies (Wang et al., 2014 Cancer Immunol Res. 2(9):846-56). In yet other embodiments, the antibody comprises a light chain constant region which is a human kappa or lambda constant region. In other embodiments, the anti-PD-1 antibody or antigen-binding portion thereof is a mAb or an antigen-binding portion thereof. In certain embodiments of any of the therapeutic methods described herein comprising administration of an anti-PD-1 antibody, the anti-PD-1 antibody is nivolumab. In other embodiments, the anti-PD-1 antibody is pembrolizumab. In other embodiments, the anti-PD-1 antibody is chosen from the human antibodies 17D8, 2D3, 4H1, 4A11, 7D3 and 5F4 described in U.S. Patent No. 8,008,449. In still other embodiments, the anti-PD-1 antibody is MEDI0608 (formerly AMP-514), AMP-224, or BGB-A317.

In embodiments, the anti-PD-1 antibody is a bispecific antibody. In embodiments, the anti-PD-1 antibody is a bispecific antibody that binds both PD-1 and LAG-3.

### 5. Anti-PD-L1 Antibodies

In certain embodiments, the present application encompasses use of an anti-PD-L1 antibody as the PD-1 pathway inhibitor. In one embodiment, the anti-PD-L1 antibody inhibits the binding of PD-L1 receptor, i.e., PD-1 to its ligand PD-L1.

Anti-human-PD-L1 antibodies (or VH and/or VL domains derived therefrom) suitable for use in the invention can be generated using methods well known in the art. Alternatively, art recognized anti-PD-L1 antibodies can be used. For example, human anti-PD-L1 antibodies disclosed in U.S. Pat. No. 7,943,743 can be used. Such anti-PD-L1 antibodies include 3G10, 12A4 (also referred to as BMS-936559), 10A5, 5F8, 10H10, 1B12, 7H1, 11E6, 12B7, and 13G4. [0110] In some embodiments, the anti-PD-L1 antibody is atezolizumab (Tecentriq or RG7446) (see, e.g., Herbst et al. (2013) J Clin Oncol 31(suppl):3000. Abstract; U.S. Patent No. 8,217,149), durvalumab (Imfinzi or MEDI4736) (Khleif (2013) In: Proceedings from the European Cancer Congress 2013; September 27-October 1, 2013; Amsterdam, The Netherlands. Abstract 802), avelumab (Bavencio). Other art recognized anti-PD-L1 antibodies which can be used include those described in, for example, U.S. Pat. Nos. 7,635,757 and 8,217,149, U.S. Publication No. 2009/0317368, and PCT Publication Nos. WO 2011/066389 and WO 2012/145493, which are herein incorporated by reference. Antibodies that compete with any of these art-recognized antibodies or inhibitors for binding to PD-L1 also can be used. Examples of anti-PD-L1 antibodies useful in the methods of the present disclosure include the antibodies disclosed in US Patent No. 9,580,507, which is herein incorporated by reference. Anti-PD-L1 human monoclonal antibodies disclosed in U.S. Patent No. 9,580,507 have been demonstrated to exhibit one or more of the following characteristics: (a) bind to human PD-L1 with a KD of 1 x 10-7 M or less, as determined by surface plasmon resonance using a Biacore biosensor system; (b) increase T-cell proliferation in a Mixed Lymphocyte Reaction (MLR) assay; (c) increase interferon-γ production in an MLR assay; (d) increase IL-2 secretion in an MLR assay; (e) stimulate antibody responses; and (f) reverse the effect of T regulatory cells on T cell effector cells and/or dendritic cells. Anti-PD-L1 antibodies usable in the present invention include monoclonal antibodies that bind specifically to human PD-L1 and exhibit at least one, in some embodiments, at least five, of the preceding characteristics.

In certain embodiments, the anti-PD-L1 antibody is BMS-936559 (formerly 12A4 or MDX-1105) (see, e.g., U.S. Patent No. 7,943,743; WO 2013/173223), which are herein incorporated by reference. In other embodiments, the anti-PD-L1 antibody is MPDL3280A (also known as RG7446 and atezolizumab) (see, e.g., Herbst et al. 2013 J Clin Oncol 31(suppl):3000; U.S. Patent No. 8,217,149), MEDI4736 (Khleif, 2013, In: Proceedings from the European Cancer Congress 2013; September 27-October 1, 2013; Amsterdam, The Netherlands. Abstract 802), or MSB0010718C (also called Avelumab; see US 2014/0341917), which are herein incorporated by reference. In certain embodiments, antibodies that cross-compete for binding to human PD-L1 with, or bind to the same epitope region of human PD-L1 as the above-references PD-L1 antibodies are mAbs. For administration to human subjects, these cross-competing antibodies can be chimeric antibodies, or can be humanized or human antibodies. Such chimeric, humanized or human mAbs can be prepared and isolated by methods well known in the art. In certain embodiments, the anti-PD-L1 antibody is selected from the group consisting of BMS-936559 (also known as 12A4, MDX-1105; see, e.g., U.S. Patent No. 7,943,743 and WO 2013/173223), atezolizumab (Roche; also known as TECENTRIQ®; MPDL3280A, RG7446; see US 8,217,149; see, also, Herbst et al. (2013) J Clin Oncol 31(suppl):3000), durvalumab (AstraZeneca; also known as IMFINZI™, MEDI-4736; see WO 2011/066389), avelumab (Pfizer; also known as BAVENCIO®, MSB-0010718C; see WO 2013/079174), STI-1014 (Sorrento; see WO2013/181634), CX-072 (Cytomx; see WO2016/149201), KN035 (3D Med/Alphamab; see Zhang et al., Cell Discov. 7:3 (March 2017), LY3300054 (Eli Lilly Co.; see, e.g., WO 2017/034916), and CK-301 (Checkpoint Therapeutics; see Gorelik et al., AACR:Abstract 4606 (Apr 2016)), which are herein incorporated by reference.

In certain embodiments, the PD-L1 antibody is atezolizumab (TECENTRIQ®). Atezolizumab is a fully humanized IgG1 monoclonal anti-PD-L1 antibody.

In certain embodiments, the PD-L1 antibody is durvalumab (IMFINZI™). Durvalumab is a human IgG1 kappa monoclonal anti-PD-L1 antibody.

In certain embodiments, the PD-L1 antibody is avelumab (BAVENCIO®). Avelumab is a human IgG1 lambda monoclonal anti-PD-L1 antibody.

In other embodiments, the anti-PD-L1 monoclonal antibody is selected from the group consisting of 28-8, 28-1, 28-12, 29-8, 5H1, and any combination thereof.

Anti-PD-L1 antibodies usable in the disclosed methods also include isolated antibodies that bind specifically to human PD-L1 and cross-compete for binding to human PD-L1 with any anti-PD-L1 antibody disclosed herein, e.g., atezolizumab, durvalumab, and/or avelumab. In some embodiments, the anti-PD-L1 antibody binds the same epitope as any of the anti-PD-L1 antibodies described herein, e.g., atezolizumab, durvalumab, and/or avelumab. The ability of antibodies to cross-compete for binding to an antigen indicates that these antibodies bind to the same epitope region of the antigen and sterically hinder the binding of other cross-competing antibodies to that particular epitope region. These cross-competing antibodies are expected to have functional properties very similar those of the reference antibody, e.g., atezolizumab and/or avelumab, by virtue of their binding to the same epitope region of PD-L1. Cross-competing antibodies can be readily identified based on their ability to cross-compete with atezolizumab and/or avelumab in standard PD-L1 binding assays such as Biacore analysis, ELISA assays or flow cytometry (see, e.g., WO 2013/173223, which is herein incorporated by reference).

In certain embodiments, the antibodies that cross-compete for binding to human PD-L1 with, or bind to the same epitope region of human PD-L1 antibody as, atezolizumab, durvalumab, and/or avelumab, are monoclonal antibodies. For administration to human subjects, these cross-competing antibodies are chimeric antibodies, engineered antibodies, or humanized or human antibodies. Such chimeric, engineered, humanized or human monoclonal antibodies can be prepared and isolated by methods well known in the art.

Anti-PD-L1 antibodies usable in the methods of the disclosed invention also include antigen-binding portions of the above antibodies. It has been amply demonstrated that the antigen-binding function of an antibody can be performed by fragments of a full-length antibody.

Anti-PD-L1 antibodies suitable for use in the disclosed methods or compositions are antibodies that bind to PD-L1 with high specificity and affinity, block the binding of PD-1, and inhibit the immunosuppressive effect of the PD-1 signaling pathway. In any of the compositions or methods disclosed herein, an anti-PD-L1 "antibody" includes an antigen-binding portion or fragment that binds to PD-L1 and exhibits the functional properties similar to those of whole antibodies in inhibiting receptor binding and upregulating the immune system. In certain embodiments, the anti-PD-L1 antibody or antigen-binding portion thereof cross-competes with atezolizumab, durvalumab, and/or avelumab for binding to human PD-L1.

Anti-PD-L1 antibodies useful for the invention include antibodies engineered starting from antibodies having one or more of the $V_H$ and/or $V_L$ sequences disclosed herein, which engineered antibodies can have altered properties from the starting antibodies. An anti-PD-L1 antibody can be engineered by a variety of modifications as described above for the engineering of modified anti-PD-1 antibodies of the invention.

## 6. Chemotherapeutic Agents

The methods of the invention features using one or more chemotherapeutic agents in combination with the LAG-3 inhibitor and the PD-1 pathway inhibitor to treat malignant tumors. In one embodiment, the chemotherapeutic agent is considered the standard of care for treatment of the malignant tumor. A "chemotherapeutic agent" is a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (*e.g.*, calicheamicin, especially calicheamicin gamma1I and calicheamicin omega1I (*see, e.g.*, Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, e.g., paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (e.g., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylomithine (DMFO); retinoids such as retinoic acid, including bexarotene (TARGRETIN®); bisphosphonates such as clodronate (for example, BONEFOS® or OSTAC®), etidronate (DIDROCAL®),

NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); BAY439006 (sorafenib; Bayer); SU-11248 (sunitinib, SUTENT®, Pfizer); perifosine, COX-2 inhibitor (e.g. celecoxib or etoricoxib), proteosome inhibitor (e.g. PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (see definition below); tyrosine kinase inhibitors (see definition below); serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; IFL, an abbreviation for a treatment regimen with irinotecan, combined with 5-fluorouracil, and leucovorin; XELOX, an abbreviation for a treatment regimen with oxaliplatin (ELOXATIN™) combined with capecitabine, SOX, an abbreviation for a treatment regimen with oxaliplatin (ELOXATIN™) combined with tegafur/gimeracit/oteracil potassium, and FOLFOX, an abbreviation for a treatment regimen with oxaliplatin (ELOXATIN™) combined with 5-FU and leucovorin. In some embodiments, the one or more chemotherapeutic agents of the methods of the invention are XELOX, FOLFOX, or SOX.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releaseing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); anti-androgens such as flutamide, nilutamide and bicalutamide; and pharmaceutically acceptable salts, acids or derivatives of any of the above; as well as combinations of two or more of the above.

## 7. Pharmaceutical Compositions

Pharmaceutical compositions suitable for administration to human patients are typically formulated for parenteral administration, e.g., in a liquid carrier, or suitable for reconstitution into liquid solution or suspension for intravenous administration.

In general, such compositions typically comprise a pharmaceutically acceptable carrier. As used herein, the term "pharmaceutically acceptable" means approved by a government regulatory agency or listed in the U.S. Pharmacopeia or another generally recognized pharmacopeia for use in animals, particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil, glycerol polyethylene glycol ricinoleate, and the like. Water or aqueous solution saline and aqueous dextrose and glycerol solutions may be employed as carriers, particularly for injectable solutions (e.g., comprising an anti-LAG-3 and/or anti-PD-1 antibody). Liquid compositions for parenteral administration can be formulated for administration by injection or continuous infusion. Routes of administration by injection or infusion include intravenous, intraperitoneal, intramuscular, intrathecal and subcutaneous. In one embodiment, the anti-LAG-3 and/or anti-PD-1 antibodies are administered intravenously (*e.g.*, in separate formulations or together (in the same formulation or in separate formulations)).

## 8. Patient Populations

Provided herein are clinical methods for treating malignant tumors (*e.g.*, advanced refractory solid tumors and hematological malignancies) in human patients using an immunotherapy disclosed herein, for example, a combination of a LAG-3 inhibitor (*e.g.*, an anti-LAG-3 antibody), a PD-1 pathway inhibitor (e.g., an anti-PD-1 antibody), and one or more chemotherapeutic agents.

Examples of malignant tumors that may be treated using the methods of the invention, include liver cancer, hepatocellular carcinoma (HCC), bone cancer, pancreatic cancer, skin cancer, oral cancer, cancer of the head or neck, breast cancer, lung cancer, small cell lung cancer, NSCLC, cutaneous or intraocular malignant melanoma, renal cancer, uterine cancer, ovarian cancer, colorectal cancer, colon cancer, rectal cancer, cancer of the anal region, gastric cancer, gastroesophageal junction cancer, testicular cancer, uterine cancer, carcinoma of the fallopian tubes, carcinoma of the endometrium, carcinoma of the cervix, carcinoma of the vagina, carcinoma of the vulva, squamous cell carcinoma of the head and neck (SCCHN), non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present invention is also applicable to treatment of metastatic cancers.

In one embodiment, the human patient suffers from a malignant tumor that is refractory to treatment with an immune checkpoint inhibitor. In another embodiment, the patient suffers from a malignant tumor that is refractory to treatment with a PD-1 inhibitor. In another embodiment, the patient suffers from a malignant tumor that is refractory to treatment with an anti-PD-1 antibody. In another embodiment, the patient suffers from a malignant tumor that is refractory to treatment with an anti-PD-L1 antibody. In some embodiments, the malignant tumor is gastric cancer or gastroesophageal junction cancer.

In one embodiment, the human patient suffers from gastric cancer or gastroesophageal junction cancer. In another embodiment, the patient suffers from gastric cancer or gastroesophageal junction cancer that is refractory to treatment with a cancer therapy. In some embodiments, the cancer therapy can be radiation therapy, surgery, chemotherapy, gene therapy, DNA therapy, viral therapy, RNA therapy, immunotherapy, bone marrow transplantation, nanotherapy, monoclonal antibody therapy, or a combination of the foregoing. The therapy may be in the form of adjuvant or neoadjuvant therapy. "Adjuvant therapy," as used herein refers to cancer treatment given after the primary treatment to lower the risk that the cancer will come back. Adjuvant therapy may include chemotherapy, radiation therapy, hormone therapy, targeted therapy, or biological therapy. Adjuvant therapy is often used after primary treatments, such as surgery or radiation. Adjuvant therapy given before the main treatment is called neoadjuvant therapy. This type of adjuvant therapy can also decrease the chance of the cancer coming back, and its often used to make the primary treatment, *e.g.*, surgery or radiation treatment, more effective in reducing tumor burden. In another embodiment, the patient suffers from gastric cancer or gastroesophageal junction cancer that is refractory to treatment with chemotherapy. In another embodiment, the patient suffers from gastric cancer or gastroesophageal junction cancer that is refractory to treatment with an immune checkpoint inhibitor. In another embodiment, the patient suffers from gastric cancer or gastroesophageal junction cancer that is refractory to treatment with a PD-1 inhibitor. In another embodiment, the patient suffers from gastric cancer or gastroesophageal junction cancer that is refractory to treatment with an anti-PD-1 antibody. In another embodiment, the patient suffers from gastric cancer or gastroesophageal junction cancer that is refractory to treatment with an anti-PD-L1 antibody.

Patients can be tested or selected for one or more of the above described clinical attributes prior to, during or after treatment.

In accordance with the methods described herein, the malignant tumors can be tested to determine LAG-3 expression. In some embodiments, the malignant tumors treated in accordance with the methods disclosed herein are LAG-3 positive tumors. In some embodiments, the malignant tumor is a LAG-3-positive gastric adenocarcinoma or gastroesophageal junction adenocarcinoma.

In some embodiments, at least about 0.5%, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, or at least about 30% of the total number of cells of a malignant tumor express LAG-3. In some embodiments, the percentage of cells that express LAG-3 is assessed by performing an assay to detect the presence of LAG-3 RNA. In further embodiments, the presence of LAG-3 RNA is detected by RT-PCR, *in situ* hybridization or RNase protection. In some embodiments, the presence of LAG-3 RNA is detected by an RT-PCR based assay. In other embodiments, the percentage of cells that express LAG-3 is assessed by performing an assay to detect the presence of LAG-3 polypeptide. In some embodiments, the presence of LAG-3 polypeptide is detected by IHC, ELISA, *in vivo* imaging, or flow cytometry. In some embodiments, LAG-3 expression is assayed by IHC.

In accordance with the methods described herein, the malignant tumors can be tested to determine LAG-3 and PD-L1 expression. In some embodiments, the malignant tumors treated in accordance with the methods disclosed herein are LAG-3 positive. PD-L1 positive tumors. In some embodiments, the malignant tumor is a LAG-3 positive, PD-L1 positive gastric adenocarcinoma or gastroesophageal junction adenocarcinoma.

In embodiments, the patient is HER2 negative. In some embodiments, the patient has a histologically- or cytologically- confirmed diagnosis of unresectable, locally advanced, or metastatic gastric cancer or gastroesophageal junction adenocarcinoma. In certain embodiments, the patient has not received prior systematic treatments. In embodiments, the patient has not received HER2 inhibitors. In particular embodiments, the patient does not have known untreated central nervous system metastases. In embodiments, the patient does not have uncontrolled or significant cardiovascular disease. In some embodiments, the patient has an ECOG performance status score of 0 or 1.

### 9. Immunotherapies

In one aspect, immunotherapies provided herein involve administration of a LAG-3 inhibitor (*e.g.,* an anti-LAG-3 antibody), a PD-1 pathway inhibitor (*e.g.,* an anti-PD-1 antibody or an anti-PD-L1 antibody), and one or more chemotherapeutic agents, to treat subjects having malignant tumors (*e.g.,* advanced refractory solid tumors or hematological malignancies).

In one embodiment, the invention provides an anti-LAG-3 antibody and an anti-PD-1 antibody in combination with chemotherapeutic agents according to a defined clinical dosage regimen, to treat subjects having a malignant tumor (*e.g.,* an advanced refractory solid tumor). In a particular embodiment, the anti-LAG-3 antibody is BMS-986016. In another embodiment, the anti-PD-1 antibody is BMS-936558. In another embodiment, dosage regimens are fixed. In another embodiment, dosage regimens are adjusted to provide the optimum desired response (*e.g.,* an effective response).

As used herein, adjunctive or combined administration (coadministration) includes simultaneous administration of the compounds in the same or different dosage form, or separate administration of the compounds (*e.g.,* sequential administration). Thus, for example, the anti-LAG-3 and anti-PD-1 antibodies can be simultaneously administered in a single formulation. Alternatively, the anti-LAG-3 and anti-PD-1 antibodies can be formulated for separate administration and are administered concurrently or sequentially (*e.g.,* one antibody is administered within about 30 minutes prior to administration of the second antibody).

For example, the anti-PD-1 antibody can be administered first followed by (*e.g.,* immediately followed by) the administration of the anti-LAG-3 antibody, or vice versa. In one embodiment, the anti-PD-1 antibody is administered prior to administration of the anti-LAG-3 antibody. In another embodiment, the anti-PD-1 antibody is administered after administration of the anti-LAG-3 antibody. In another embodiment, the anti-LAG-3 antibody and anti-PD-1 antibody are administered concurrently. Such concurrent or sequential administration preferably results in both antibodies being simultaneously present in treated patients.

### 10. Treatment Protocols

In one aspect, suitable treatment protocols for treating a malignant tumor in a human patient include administering to the patient an effective amount of a LAG3 inhibitor (*e.g.,* an anti-LAG-3 antibody), a PD-1 pathway inhibitor (*e.g.,* an anti-PD-1 antibody), and one or more chemotherapeutic agents.

In some embodiments, a suitable treatment protocol for treating a malignant tumor in a human patient include, for example, administering to the patient an effective amount of each of:

(a) an anti-LAG-3 antibody, such as one comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,

(b) an anti-PD-1 antibody, such as one comprising CDR1, CDR2 and CDR3 domains of the heavy chain variable region having the sequence set forth in SEQ ID NO:19, and CDR1, CDR2 and CDR3 domains of the light chain variable region having the sequence set forth in SEQ ID NO:21, and

(c) one or more chemotherapeutic agents,

wherein the method comprises at least one administration cycle, wherein the cycle is a period of six weeks, wherein for each of the at least one cycles, at least two doses of the anti-LAG-3 antibody are administered at a dose of about 1, 3, 10, 20, 50, 80, 100, 120, 130, 150, 160, 180, 200, 240 or 280 mg and at least two doses of the anti-PD-1 antibody are administered at a dose of about 50, 80, 100, 130, 150, 180, 200, 240, 280, 320, 360, 400, 440, or 480 mg. In certain embodiments, the anti-LAG-3 antibody is administered at a dose of about 320, 360, 400, 440, 480, 520, 560, 600, 640, 680, 720, 760, 800, 840, 880, 920, 960, or 1000 mg. In certain embodiments, the anti-LAG-3 antibody is administered at a dose of about 1040, 1080, 1120, 1160, 1200, 1240, 1280, 1320, 1360, 1400, 1440, 1480, 1520, 1560, 1600, 1640, 1680, 1720, 1760, 1800, 1840, 1880, 1920, 1960, or 2000 mg. In particular embodiments, the anti-LAG-3 antibody is administered at a dose of about 480 mg. In another embodiment, four doses of the anti-LAG-3 antibody are administered at a dose of 0.01, 0.03, 0.25, 0.1, 0.3, 1 or 3, 5, 8 or 10 mg/kg body weight and four doses of the anti-PD-1 antibody are administered at a dose of 0.1, 0.3, 1, 3, 5, 8 or 10 mg/kg body weight.

In certain embodiments, the anti-LAG-3 antibody is administered at a dose of about 300 mg to about 500 mg once every three weeks. In some embodiments, the anti-LAG-3 antibody is administered at a dose of about 400 mg once every three weeks. In some embodiments, the anti-LAG-3 antibody is administered at a dose of about 700 mg to about 900 mg once every four weeks.

In a further embodiment, one or more chemotherapeutic agents are administered. In one embodiment, at least one chemotherapeutic agent is administered intravenously. In one embodiment, at least one chemotherapeutic agent is administered orally.

In one embodiment, the one or more chemotherapeutic agents are administered using a body surface area-based dosing.

In one embodiment, the anti-LAG-3 antibody and anti-PD-1 antibody are administered at the following doses:

> (a) 120 mg anti-LAG-3 antibody and 360 mg of anti-PD-1 antibody; or
> (b) 160 mg anti-LAG-3 antibody and 480 mg of anti-PD-1 antibody.

In one embodiment, the tumor is gastric or gastroesophageal junction cancer.

In another embodiment, the amount of the anti-LAG-3 and/or anti-PD-1 antibodies administered is constant for each dose. In another embodiment, the amount of antibody administered varies with each dose. For example, the maintenance (or follow-on) dose of the antibody can be higher or the same as the loading dose which is first administered. In another embodiment, the maintenance dose of the antibody can be lower or the same as the loading dose.

In another embodiment, the anti-LAG-3 and anti-PD-1 antibodies are formulated for intravenous administration. In one embodiment, the anti-LAG-3 antibody and anti-PD-1 antibody are administered on Days 1 and 22 of each cycle. In one embodiment, one or more chemotherapeutic agent is administered on Days 1 and 22 of each cycle. In one embodiment, one or more chemotherapeutic agent is administered at least once daily.

In another embodiment, a cycle of administration is six weeks, which can be repeated, as necessary. In another embodiment, the treatment consists of up to 12 cycles.

In one embodiment, the anti-LAG-3 antibody and anti-PD-1 antibody are administered at the following doses: (a) 120 mg anti-LAG-3 antibody and 360 mg of anti-PD-1 antibody, on days 1 and 22 of each treatment cycle every 6 weeks; and oxaliplatin and capecitabine (XELOX) are administered. In a further embodiment, oxaliplatin 130 mg/m$^2$ is administered on days 1 and 22 of each treatment cycle every 6 weeks and capecitabine 1000 mg/m$^2$ is administered twice daily on days 1 to 14 and days 22 to 35 of each treatment cycle every 6 weeks.

In one embodiment, the anti-LAG-3 antibody and anti-PD-1 antibody are administered at the following doses: (a) 160 mg anti-LAG-3 antibody and 480 mg of anti-PD-1 antibody, on days 1 and 29 of every odd numbered cycle (cycle 1, 3, 5, etc.) and day 15 of every even numbered cycle (cycle 2, 4, 6, etc.) and oxaliplatin, leucovorin, fluorouracil (FOLFOX) are administered. In a further embodiment, oxaliplatin 85 mg/m$^2$, leucovorin 400 mg/m$^2$, and fluorouracil 400 mg/m$^2$ is administered on days 1, 15, and 29 of each treatment cycle every 6 weeks and fluorouracil 1200 mg/m$^2$ on days 1 & 2, 15 & 16, 29 and 30 of each treatment cycle every 6 weeks.

In one embodiment, the anti-LAG-3 antibody and anti-PD-1 antibody are administered at the following doses: (a) 120 mg anti-LAG-3 antibody and 360 mg of anti-PD-1 antibody, on days 1 and 22 of each treatment cycle every 6 weeks; and oxaliplatin and tegafur/gimeracil/oteracil (Oral S-1) (SOX) are administered. In a further embodiment, oxaliplatin 130 mg/m$^2$ is administered on days 1 and 22 of each treatment cycle every 6 weeks and Oral S-1 is administered twice daily on days 1 to 14 and days 22 to 35 of each treatment cycle every 6 weeks. In a further embodiment, S-1 dose is calculated according to body surface area (BSA, mg/m2/dose): BSA <1.25 m$^2$, 40 mg/dose; $\geq$ 1.25 and < 1.5 m$^2$, 50 mg/dose; $\geq$ 1.5 m$^2$, 60 mg/dose.

In another embodiment, the anti-LAG-3 antibody, the anti-PD-1 antibody, and chemotherapeutic agents are administered as a first line of treatment (e.g., the initial or first treatment). In another embodiment, the anti-LAG-3 antibody, the anti-PD-1 antibody, and chemotherapeutic agents are administered as a second line of treatment (e.g., after the initial or first treatment, including after relapse and/or where the first treatment has failed).

In one embodiment, the anti-LAG-3 antibody is BMS-986016 and the anti-PD-1 antibody is nivolumab. In one embodiment, the anti-LAG-3 antibody is MK-4280 and the anti-PD-1 antibody is pembrolizumab. In one embodiment, the anti-LAG-3 antibody is REGN3767 and the anti-PD-1 antibody is REGN2810. In one embodiment, the anti-LAG-3 antibody is LAG525 and the anti-PD-1 antibody is PDR001.

In one embodiment, the patient is administered an effective amount of : (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and (c) one or more chemotherapeutic agents selected from the group consisting of XELOX, FOLFOX, and SOX. In embodiments, the method is administered to a patient that has not received prior therapy (e.g., first line therapy). In some embodiments, the prior therapy is a HER2 inhibitor. In particular embodiments, the anti-LAG-3 antibody and the anti-PD-1 antibody are administered as a fixed dose combination. In embodiments, the patent has recurrent, locally advanced or metastatic gastric cancer or gastoesophageal adenocarcinoma.

In another aspect, the invention features any of the aforementioned embodiments, wherein the anti-PD-1 antibody is replaced by, or combined with, an anti-PD-L1 or anti-PD-L2 antibody.

## 11. Outcomes

Patients treated according to the methods disclosed herein preferably experience improvement in at least one sign of cancer. In one embodiment, improvement is measured by a reduction in the quantity and/or size of measurable tumor lesions. In another embodiment, lesions can be measured on chest x-rays or CT or MRI films. In another embodiment, cytology or histology can be used to evaluate responsiveness to a therapy.

In one embodiment, the patient treated exhibits a complete response (CR), a partial response (PR), stable disease (SD), immune-related complete disease (irCR), immune-related partial response (irPR), or immune-related stable disease (irSD). In another embodiment, the patient treated experiences tumor shrinkage and/or decrease in growth rate, *i.e.*, suppression of tumor growth. In another embodiment, unwanted cell proliferation is reduced or inhibited. In yet another embodiment, one or more of the following can occur: the number of cancer cells can be reduced; tumor size can be reduced; cancer cell infiltration into peripheral organs can be inhibited, retarded, slowed, or stopped; tumor metastasis can be slowed or inhibited; tumor growth can be inhibited; recurrence of tumor can be prevented or delayed; one or more of the symptoms associated with cancer can be relieved to some extent.

In other embodiments, administration of effective amounts of the anti-LAG-3 antibody, anti-PD-1 antibody, and one or more chemotherapeutic agents according to any of the methods provided herein produces at least one therapeutic effect selected from the group consisting of reduction in size of a tumor, reduction in number of metastatic lesions appearing over time, complete remission, partial remission, or stable disease.

In still other embodiments, the methods of treatment produce a clinical benefit rate (CBR=CR+PR+SD$\geq$6 months) better than that achieved by a method of treatment that does not comprise a step of (i) determining the level of LAG-3 expression in a tumor sample prior to treatment, (ii) selecting a LAG-3 positive tumor for treatment, (iii) treating a tumor that has been identified as LAG-3 positive prior to treatment, or (iv) any combinations thereof. In other embodiments, the improvement of clinical benefit rate is about 20% 20%, 30%, 40%, 50%, 60%, 70%, 80% or more compared to a method of treatment that does not comprise a step of (i) determining the level of LAG-3 expression in a tumor sample prior to treatment, (ii) selecting a LAG-3 positive tumor for treatment, (iii) treating a tumor that has been identified as LAG-3 positive prior to treatment, or (iv) any combinations thereof.

In still other embodiments, the methods of treatment produce an objective response rate (ORR=CR+PR) of at least about 15%, at least about 20%, at least about 25%, at least about 30%, at least about 40%, at least about 50%, at least about 60%, at least about 70%, at least about 80%, at least about 90%, or about 100%. In one embodiment, the methods of treatment produce an objective response rate of at least about 15%, wherein the malignant tumor is a LAG-3 positive melanoma that is resistant to treatment with an anti-PD-1 or anti-PD-L1antibody. In some embodiments, the median duration of response is $\geq$ 3 month, $\geq$ 6 month, $\geq$ 12 month, or $\geq$ 18 month. In one embodiment, the median duration of response is $\geq$ 6 month. In some embodiments, the frequency of patients with duration of response $\geq$ 6 month is at least about 30%, at least about 40%, at least about 50%, at least about 60%, at least about 70%, at least about 80%, at least about 90%, at least about 95%, at least about 99% or 100%.

In still other embodiments, the methods of treatment produce an objective response rate (ORR=CR+PR) better than that achieved by a method of treatment that does not comprise a step of (i) determining the level of LAG-3 expression in a tumor sample prior to treatment, (ii) selecting a LAG-3 positive tumor for treatment, (iii) treating a tumor that has been identified as LAG-3 positive prior to treatment, or (iv) any combinations thereof. In other embodiments, the improvement of objective response rate is about 20% 20%, 30%, 40%, 50%, 60%, 70%, 80% or more compared to a method of treatment that does not comprise a step of (i) determining the level of LAG-3 expression in a tumor sample prior to treatment, (ii) selecting a LAG-3 positive tumor for treatment, (iii) treating a tumor that has been identified as LAG-3 positive prior to treatment, or (iv) any combinations thereof. In some embodiments, the median duration of response is $\geq$ 3 month, $\geq$ 6 month, $\geq$ 12 month, or $\geq$ 18 month. In one embodiment, the median duration of response is $\geq$ 6 month.

In still other embodiments, the methods of treatment produce a disease control rate (DRR=CR+PR+SD) of at least about 20%, at least about 30%, at least about 40%, at least about 50%, at least about 60%, at least about 70%, at least about 80%, at least about 90%, at least about 95%, at least about 99% or about 100%. In one embodiment, the methods of treatment produce a disease control rate of at least about 70%, wherein the malignant tumor is a LAG-3 positive melanoma that is resistant to treatment with an anti-PD-1 or anti-PD-L1 antibody. In some embodiments, the median duration of response is ≥ 3 month, ≥ 6 month, ≥ 12 month, or ≥ 18 month. In one embodiment, the median duration of response is ≥ 6 month. In some embodiments, the frequency of patients with duration of response ≥ 6 month is at least about 30%, at least about 40%, at least about 50%, at least about 60%, at least about 70%, at least about 80%, at least about 90%, at least about 95%, at least about 99% or 100%.

In still other embodiments, the methods of treatment produce a disease control rate (DRR=CR+PR+SD) better than that achieved by a method of treatment that does not comprise a step of (i) determining the level of LAG-3 expression in a tumor sample prior to treatment, (ii) selecting a LAG-3 positive tumor for treatment, (iii) treating a tumor that has been identified as LAG-3 positive prior to treatment, or (iv) any combinations thereof. In other embodiments, the improvement of disease control rate is about 20% 20%, 30%, 40%, 50%, 60%, 70%, 80% or more compared to a method of treatment that does not comprise a step of (i) determining the level of LAG-3 expression in a tumor sample prior to treatment, (ii) selecting a LAG-3 positive tumor for treatment, (iii) treating a tumor that has been identified as LAG-3 positive prior to treatment, or (iv) any combinations thereof. In some embodiments, the median duration of response is ≥ 3 month, ≥ 6 month, ≥ 12 month, or ≥ 18 month. In one embodiment, the median duration of response is ≥ 6 month.

## 12. Kits and Unit Dosage Forms

Also within the scope of the present invention are diagnostic kits comprising an anti-LAG-3 antibody for assaying LAG-3 expression as a biomarker for screening patients for the immunotherapy or for predicting the efficacy of the immunotherapy. Kits typically include a label indicating the intended use of the contents of the kit and instructions for use. The term "label" includes any writing, or recorded material supplied on or with the kit, or which otherwise accompanies the kit. In certain embodiments of a diagnostic kit, a first anti-LAG-3 antibody for assaying, detecting, and/or quantifying LAG-3 expression is co-packaged with at least one therapeutic antibody (*e.g.*, a second anti-LAG-3 antibody and an anti-PD-1 antibody) for the treatment of a LAG-3 positive tumor. In some embodiments, the kit further comprises an anti-PD-L1 antibody for assaying, detecting, and/or quantifying PD-L1 expression as a biomarker for predicting the efficacy of the immunotherapy. In one embodiment, the immunotherapy comprises administering to the patient a therapeutically effective amount of a LAG-3 inhibitor (*e.g.*, anti-LAG-3 antibody), a PD-1 pathway inhibitor (*e.g.*, anti-PD1 antibody), and one or more chemotherapeutic agents.

In certain embodiments, the diagnostic kit comprises an anti-human LAG-3 monoclonal antibody for assaying, detecting, and/or quantifying LAG-3 expression. *See, e.g.*, J. Matsuzaki, et al.; PNAS 107, 7875 (2010).

Also provided herein are therapeutic kits which include a pharmaceutical composition containing an anti-LAG-3 antibody, such as BMS-986016, an anti-PD-1 antibody, such as nivolumab, and one or more chemotherapeutic agents, in a therapeutically effective amount adapted for use in the preceding methods. In certain embodiments of a therapeutic kit, the anti-LAG-3 antibody is co-packaged with an anti-PD-1 antibody in unit dosage form. The kits optionally also can include instructions, *e.g.*, comprising administration schedules, to allow a practitioner (*e.g.*, a physician, nurse, or patient) to administer the composition contained therein to administer the composition to a patient having cancer (*e.g.*, a solid tumor). The kit also can include a syringe.

Optionally, the diagnostic and/or therapeutic kits include multiple packages of the single-dose pharmaceutical compositions each containing an effective amount of the anti-LAG-3 or anti-PD-1 antibody for a single administration in accordance with the methods provided above. Instruments or devices necessary for administering the pharmaceutical composition(s) also may be included in the kits. For instance, a kit may provide one or more pre-filled syringes containing an amount of the anti-LAG-3 or anti-PD-1 antibody.

In one embodiment, the present invention provides a kit for treating a patient afflicted with a malignant tumor, the kit, for example, comprising:

(a) a dose of an anti-LAG-3 antibody, such as one comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5;

(b) a dose of an anti-PD-1 antibody, such as one comprising CDR1, CDR2 and CDR3 domains of the heavy chain variable region having the sequence set forth in SEQ ID NO:19, and CDR1, CDR2 and CDR3 domains of the light chain variable region having the sequence set forth in SEQ ID NO:21;

(c) one or more chemotherapeutic agents; and

(d) instructions for using the anti-LAG-3 antibody, anti-PD-1 antibody, and one or more chemotherapeutic agents in the methods described herein.

In some embodiments, the malignant tumor is a gastric cancer or a gastroesophageal junction cancer.

The present invention is further illustrated by the following examples which should not be construed as further limiting. The contents of all references cited throughout this application are expressly incorporated herein by reference.

EXAMPLES

**EXAMPLE 1**

Efficacy of Anti-Lymphocyte Activation Gene-3 Antibody (Anti-LAG-3; BMS-986016) in Combination With Nivolumab and Chemotherapy in Patients With Advanced or Metastatic Gastric Cancer or Gastroesophageal Junction Cancer

The purpose of this study is to evaluate the combination of BMS-986016 (relatlimab), Nivolumab, and chemotherapy in the treatment of gastric or gastroesophageal junction cancer.

Patients are selected based on the following eligibility criteria: (1) having advanced or metastatic gastric or gastroesophageal junction cancer; (2) no prior systemic treatment; (3) Eastern Cooperative Oncology Group PS 0-1; (4) HER2 negative status; and (5) LAG-3 positive tumor as determined by IHC. During the treatment phase, patients will receive BMS-986016 and nivolumab, in combination with a choice of chemotherapy (XELOX, FOLFOX, or SOX).

Patients assigned to XELOX will receive:

- relatlimab 120 mg/nivolumab 360 mg administered IV over 60 minutes on Days 1 and 22 of each treatment cycle every 6 weeks, and
- Oxaliplatin 130 mg/m$^2$ administered IV on Days 1 and 22 of each treatment cycle every 6 weeks, and
- Capecitabine 1000 mg/m$^2$ administered orally twice daily on Days 1 to 14 and Days 22 to 35 of each treatment cycle every 6 weeks.

Patients assigned to FOLFOX will receive:

- relatlimab 160 mg/nivolumab 480 mg administered IV over 60 minutes on Days 1 and 29 of every odd numbered Cycle (Cycle 1, 3, 5, etc) and Day 15 of every even numbered Cycle (Cycle 2, 4, 6, etc), and
- Oxaliplatin 85 mg/m$^2$, leucovorin 400 mg/m$^2$, and fluorouracil 400 mg/m$^2$, administered IV on Days 1, 15, and 29 of each treatment cycle every 6 weeks, and fluorouracil 1200 mg/m$^2$ IV continuous infusion over 24 hours daily (or per local standard) on Days 1 & 2, 15 & 16, and 29 and 30 of each treatment cycle every 6 weeks.

Patients assigned to SOX will receive:

- relatlimab 120 mg/nivolumab 360 mg administered IV over 60 minutes Days 1 and 22 of each treatment cycle every 6 weeks, and
- Oxaliplatin 130 mg/m2 administered IV on Days 1 and 22 of each treatment cycle every 6 weeks, and
- Oral S-1 (tegafur/gimeracil/oteracil) twice daily on Days 1 to 14 and Days 22 to 35 of each treatment cycle, every 6 weeks. S-1 dose as calculated according to body surface area (BSA, mg/m2/dose): BSA <1.25 m$^2$, 40 mg/dose; $\geq$ 1.25 and <1.5 m$^2$, 50 mg/dose; $\geq$1.5m$^2$, 60 mg/dose.

SEQUENCES

SEQ ID NO:1 Heavy Chain Amino Acid Sequence; Anti-LAG-3 mAb (BMS-986016)

```
QVQLQQWGAGLLKPSETLSLTCAVYGGSFSDYYWNWIRQPPGKGLEWIGEINHRGSTNSNPSLKS
RVTLSLDTSKNQFSLKLRSVTAADTAVYYCAFGYSDYEYNWFDPWGQGTLVTVSSASTKGPSVFP
LAPCSRSTSESTAALGCLVKDYFPEPVTVSWNSGALTSGVHTFPAVLQSSGLYSLSSVVTVPSSS
LGTKTYTCNVDHKPSNTKVDKRVESKYGPPCPPCPAPEFLGGPSVFLFPPKPKDTLMISRTPEVT
CVVVDVSQEDPEVQFNWYVDGVEVHNAKTKPREEQFNSTYRVVSVLTVLHQDWLNGKEYKCKVSN
KGLPSSIEKTISKAKGQPREPQVYTLPPSQEEMTKNQVSLTCLVKGFYPSDIAVEWESNGQPENN
YKTTPPVLDSDGSFFLYSRLTVDKSRWQEGNVFSCSVMHEALHNHYTQKSLSLSLGK
```

SEQ ID NO:2 Light Chain Amino Acid Sequence; Anti-LAG-3 mAb (BMS-986016)

EIVLTQSPATLSLSPGERATLSCRASQSISSYLAWYQQKPGQAPRLLIYDASNRATGIPARFSGS
GSGTDFTLTISSLEPEDFAVYYCQQRSNWPLTFGQGTNLEIKRTVAAPSVFIFPPSDEQLKSGTA
SVVCLLNNFYPREAKVQWKVDNALQSGNSQESVTEQDSKDSTYSLSSTLTLSKADYEKHKVYACE
VTHQGLSSPVTKSFNRGEC

SEQ ID NO:3 Heavy Chain Variable Region (VH) Amino Acid Sequence; Anti-LAG-3 mAb (BMS-986016)

QVQLQQWGAGLLKPSETLSLTCAVYGGSFSDYYWNWIRQPPGKGLEWIGEINHRGSTNSNPSLKS
RVTLSLDTSKNQFSLKLRSVTAADTAVYYCAFGYSDYEYNWFDPWGQGTLVTVSS

SEQ ID NO:4 Heavy Chain Variable Region (VH) Nucleotide Sequence; Anti-LAG-3 mAb (BMS-986016)

caggtgcagctacagcagtggggcgcaggactgttgaagccttcggagaccctgtccctcacctg
cgctgtctatggtgggtccttcagtgattactactggaactggatccgccagcccccagggaagg
ggctggagtggattggggaaatcaatcatcgtggaagcaccaactccaacccgtccctcaagagt
cgagtcaccctatcactagcacgtccaagaaccagttctccctgaagctgaggtctgtgaccgc
cgcggacacggctgtgtattactgtgcgtttggatatagtgactacgagtacaactggttcgacc
cctggggccagggaaccctggtcaccgtctcctca

SEQ ID NO:5 Light Chain Variable Region (VL) Amino Acid Sequence; Anti-LAG-3 mAb (BMS-986016)

EIVLTQSPATLSLSPGERATLSCRASQSISSYLAWYQQKPGQAPRLLIYDASNRATGIPARFSGS
GSGTDFTLTISSLEPEDFAVYYCQQRSNWPLTFGQGTNLEIK

SEQ ID NO:6 Light Chain Variable Region (VL) Nucleotide Sequence; Anti-LAG-3 mAb (BMS-986016)

gaaattgtgttgacacagtctccagccaccctgtctttgtctccaggggaaagagccaccctctc
ctgcagggccagtcagagtattagcagctacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagcgtagcaactggcctctcacttttggccaggggaccaacctggagatcaaa

SEQ ID NO:7 Heavy Chain CDR1 Amino Acid Sequence; Anti-LAG-3 mAb (BMS-986016)
DYYWN

SEQ ID NO:8 Heavy Chain CDR2 Amino Acid Sequence; Anti-LAG-3 mAb (BMS-986016)
EINHRGSTNSNPSLKS

SEQ ID NO:9 Heavy Chain CDR3 Amino Acid Sequence; Anti-LAG-3 mAb (BMS-986016)
GYSDYEYNWFDP

SEQ ID NO:10 Light Chain CDR1 Amino Acid Sequence; Anti-LAG-3 mAb (BMS-986016)
RASQSISSYLA

SEQ ID NO:11 Light Chain CDR2 Amino Acid Sequence; Anti-LAG-3 mAb (BMS-986016)
DASNRAT

SEQ ID NO:12 Light Chain CDR3 Amino Acid Sequence; Anti-LAG-3 mAb (BMS-986016)
QQRSNWPLT

SEQ ID NO:13 Heavy Chain Amino Acid Sequence; Anti-PD-1 mAb (BMS936558)

QVQLVESGGGVVQPGRSLRLDCKASGITFSNSGMHWVRQAPGKGLEWVAVIWYDGSKRYYADSVK
GRFTISRDNSKNTLFLQMNSLRAEDTAVYYCATNDDYWGQGTLVTVSSASTKGPSVFPLAPCSRS
TSESTAALGCLVKDYFPEPVTVSWNSGALTSGVHTFPAVLQSSGLYSLSSVVTVPSSSLGTKTYT
CNVDHKPSNTKVDKRVESKYGPPCPPCPAPEFLGGPSVFLFPPKPKDTLMISRTPEVTCVVVDVS
QEDPEVQFNWYVDGVEVHNAKTKPREEQFNSTYRVVSVLTVLHQDWLNGKEYKCKVSNKGLPSSI
EKTISKAKGQPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGK

SEQ ID NO:14 Light Chain Amino Acid Sequence; Anti-PD-1 mAb (BMS936558)

EIVLTQSPATLSLSPGERATLSCRASQSVSSYLAWYQQKPGQAPRLLIYDASNRATGIPARFSGS
GSGTDFTLTISSLEPEDFAVYYCQQSSNWPRTFGQGTKVEIKRTVAAPSVFIFPPSDEQLKSGTA
SVVCLLNNFYPREAKVQWKVDNALQSGNSQESVTEQDSKDSTYSLSSTLLSKADYEKHKVYACEV
THQGLSSPVTKSFNRGEC

SEQ ID NO:15 Heavy Chain Variable Region (VH) Amino Acid Sequence; Anti-PD-1 mAb (BMS936558)

QVQLVESGGGVVQPGRSLRLDCKASGITFSNSGMHWVRQAPGKGLEWVAVIWYDGSKRYYADSVK
GRFTISRDNSKNTLFLQMNSLRAEDTAVYYCATNDDYWGQGTLVTVSS

SEQ ID NO:16 Heavy Chain Variable Region (VH) Nucleotide Sequence; Anti-PD-1 mAb (BMS936558)

caggtgcagctggtggagtctggggggaggcgtggtccagcctgggaggtccctgagactcgactg
taaagcgtctggaatcaccttcagtaactctggcatgcactgggtccgccaggctccaggcaagg
ggctggagtgggtggcagttatttggtatgatggaagtaaaagatactatgcagactccgtgaag
ggccgattcaccatctccagagacaattccaagaacacgctgtttctgcaaatgaacagcctgag
agccgaggacacggctgtgtattactgtgcgacaaacgacgactactggggccagggaaccctgg
tcaccgtctcctca

SEQ ID NO:17 Light Chain Variable Region (VL) Amino Acid Sequence; Anti-PD-1 mAb (BMS936558)

EIVLTQSPATLSLSPGERATLSCRASQSVSSYLAWYQQKPGQAPRLLIYDASNRATGIPARFSGS
GSGTDFTLTISSLEPEDFAVYYCQQSSNWPRTFGQGTKVEIK

SEQ ID NO:18 Light Chain Variable Region (VL) Nucleotide Sequence; Anti-PD-1 mAb (BMS936558)

gaaattgtgttgacacagtctccagccaccctgtctttgtctccaggggaaagagccaccctctc
ctgcagggccagtcagagtgttagtagttacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagagtagcaactggcctcggacgttcggccaagggaccaaggtggaaatcaaa

SEQ ID NO:19 Heavy Chain CDR1 Amino Acid Sequence; Anti-PD-1 mAb (BMS936558)
NSGMH

SEQ ID NO:20 Heavy Chain CDR2 Amino Acid Sequence; Anti-PD-1 mAb (BMS936558)
VIWYDGSKRYYADSVKG

SEQ ID NO:21 Heavy Chain CDR3 Amino Acid Sequence; Anti-PD-1 mAb (BMS936558)
NDDY

SEQ ID NO:22 Light Chain CDR1 Amino Acid Sequence; Anti-PD-1 mAb (BMS936558)
RASQSVSSYLA

SEQ ID NO:23 Light Chain CDR2 Amino Acid Sequence; Anti-PD-1 mAb (BMS936558)
DASNRAT

SEQ ID NO:24 Light Chain CDR3 Amino Acid Sequence; Anti-PD-1 mAb (BMS936558)
QQSSNWPRT

SEQ ID NO:25 Heavy Chain Nucleotide Sequence; Anti-LAG-3 mAb (BMS-986016)

```
caggtgcagctacagcagtggggcgcaggactgttgaagccttcggagaccctgtccctcacctg
cgctgtctatggtgggtccttcagtgattactactggaactggatccgccagcccccagggaagg
ggctggagtggattggggaaatcaatcatcgtggaagcaccaactccaacccgtccctcaagagt

cgagtcaccctatcactagacacgtccaagaaccagttctccctgaagctgaggtctgtgaccgc
cgcggacacggctgtgtattactgtgcgtttggatatagtgactacgagtacaactggttcgacc
cctggggccagggaaccctggtcaccgtctcctcagctagcaccaagggcccatccgtcttcccc
ctggcgccctgctccaggagcacctccgagagcacagccgccctgggctgcctggtcaaggacta
cttccccgaaccggtgacggtgtcgtggaactcaggcgccctgaccagcggcgtgcacaccttcc
cggctgtcctacagtcctcaggactctactccctcagcagcgtggtgaccgtgccctccagcagc
ttgggcacgaagacctacacctgcaacgtagatcacaagcccagcaacaccaaggtggacaagag
agttgagtccaaatatggtcccccatgcccaccatgcccagcacctgagttcctggggggaccat
cagtcttcctgttccccccaaaacccaaggacactctcatgatctcccggacccctgaggtcacg
tgcgtggtggtggacgtgagccaggaagaccccgaggtccagttcaactggtacgtggatggcgt
ggaggtgcataatgccaagacaaagccgcgggaggagcagttcaacagcacgtaccgtgtggtca
gcgtcctcaccgtcctgcaccaggactggctgaacggcaaggagtacaagtgcaaggtctccaac
aaaggcctcccgtcctccatcgagaaaaccatctccaaagccaaagggcagccccgagagccaca
ggtgtacaccctgcccccatcccaggaggagatgaccaagaaccaggtcagcctgacctgcctgg
tcaaaggcttctaccccagcgacatcgccgtggagtgggagagcaatgggcagccggagaacaac
tacaagaccacgcctcccgtgctggactccgacggctccttcttcctctacagcaggctaaccgt
ggacaagagcaggtggcaggaggggaatgtcttctcatgctccgtgatgcatgaggctctgcaca
accactacacacagaagagcctctccctgtctctgggtaaatga
```

SEQ ID NO:26 Light Chain Nucleotide Sequence; Anti-LAG-3 mAb (BMS-986016)

```
gaaattgtgttgacacagtctccagccaccctgtctttgtctccaggggaaagagccaccctctc
ctgcagggccagtcagagtattagcagctacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagcgtagcaactggcctctcacttttggccaggggaccaacctggagatcaaacgta
cggtggctgcaccatctgtcttcatcttcccgccatctgatgagcagttgaaatctggaactgcc
tctgttgtgtgcctgctgaataacttctatcccagagaggccaaagtacagtggaaggtggataa
cgccctccaatcgggtaactcccaggagagtgtcacagagcaggacagcaaggacagcacctaca
gcctcagcagcaccctgacgctgagcaaagcagactacgagaaacacaaagtctacgcctgcgaa
gtcacccatcagggcctgagctcgcccgtcacaaagagcttcaacaggggagagtgttag
```

The following numbered clauses, describing aspects of the invention, are part of the description:

1. A method of inhibiting the growth of a malignant tumor in a human patient, the method comprising administering to the patient an effective amount of each of:

(a) a LAG-3 antagonist;
(b) a PD-1 pathway inhibitor; and
(c) one or more chemotherapeutic agents;

wherein the patient's tumor associated immune cells express LAG-3.

2. A method of treating cancer in a human patient, the method comprising administering to the patient an effective amount of each of:

> (a) a LAG-3 antagonist;
> (b) a PD-1 pathway inhibitor; and
> (c) one or more chemotherapeutic agents;

wherein the patient's tumor associated immune cells express LAG-3.

3. A method of inhibiting the growth of a malignant tumor in a human patient, the method comprising administering to the patient an effective amount of each of:

> (a) a LAG-3 antagonist;
> (b) a PD-1 pathway inhibitor; and
> (c) one or more chemotherapeutic agents.

4. A method of treating cancer in a human patient, the method comprising administering to the patient an effective amount of each of:

> (a) a LAG-3 antagonist;
> (b) a PD-1 pathway inhibitor; and
> (c) one or more chemotherapeutic agents.

5. The method of any one of clauses 1-4, wherein the malignant tumor is selected from the group consisting of a liver cancer, bone cancer, pancreatic cancer, skin cancer, oral cancer, cancer of the head or neck, breast cancer, lung cancer, including small cell and non-small cell lung cancer, cutaneous or intraocular malignant melanoma, renal cancer, uterine cancer, ovarian cancer, colorectal cancer, colon cancer, rectal cancer, cancer of the anal region, gastric cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, cancers of the childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combination thereof.

6. The method of any one of clauses 1-5, wherein the malignant tumor is a gastric cancer or gastroesophageal junction cancer.

7. The method of clause 6, wherein the gastric cancer is an adenocarcinoma, lymphoma, gastrointestinal stromal tumor, or carcinoid tumor.

8. The method of any of clauses 1-5, wherein the malignant tumor is chosen from melanoma, non-small cell lung cancer (NSCLC), human papilloma virus (HPV)-related tumor, bladder cancer, head and neck squamous cell carcinoma, renal cell cancer, and gastric adenocarcinoma.

9. The method of any one of clauses 1-8, wherein the LAG-3 antagonist is an anti-LAG-3 antibody.

10. The method of clause 9, wherein the anti-LAG-3 antibody is a full-length antibody.

11. The method of clause 10, wherein the antibody is a monoclonal, human, humanized, chimeric, or multispecific antibody.

12. The method of clause 11, wherein the multispecific antibody is a dual-affinity re-targeting antibody (DART), a DVD-Ig, or bispecific antibody.

13. The method of clause 9, wherein the antibody is a F(ab')$_2$ fragment, a Fab' fragment, a Fab fragment, a Fv fragment, a scFv fragment, a dsFv fragment, a dAb fragment, or a single chain binding polypeptide.

14. The method of clause 9, wherein the anti-LAG-3 antibody is BMS-986016, IMP731 (H5L7BW), MK-4280 (28G-10), REGN3767, GSK2831781, humanized BAP050, IMP-701 (LAG-5250), aLAG3(0414), aLAG3(0416), Sym022, TSR-033, TSR-075, XmAb22841, BI 754111, MGD013, AVA-017, P 13B02-30, or FS-118.

15. The method of any of clauses 1-8, wherein the LAG-3 antagonist is IMP321.

16. The method of any of clauses 9-13, wherein the anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5.

17. The method of any of clauses 1-14, wherein the wherein the anti-LAG-3 antibody comprises

(a) a heavy chain variable region CDR1 comprising the sequence set forth in SEQ ID NO:7;
(b) a heavy chain variable region CDR2 comprising the sequence set forth in SEQ ID NO:8;
(c) a heavy chain variable region CDR3 comprising the sequence set forth in SEQ ID NO:9;
(d) a light chain variable region CDR1 comprising the sequence set forth in SEQ ID NO:10;
(e) a light chain variable region CDR2 comprising the sequence set forth in SEQ ID NO:11; and
(f) a light chain variable region CDR3 comprising the sequence set forth in SEQ ID NO:12.

18. The method of any of clauses 1-11, wherein the anti-LAG-3 antibody comprises heavy and light chain variable regions comprising the sequences set forth in SEQ ID NOs:3 and 5, respectively.

19. The method of any of clauses 1-11, wherein the anti-LAG-3 antibody comprises heavy and light chains comprising the sequences set forth in SEQ ID NOs: 1 and 2, respectively.

20. The method of any of clauses 1-19, wherein the PD-1 pathway inhibitor is an anti-PD-1 or an anti-PD-L1 antibody.

21. The method of clause 20, wherein the anti-PD-1 antibody is selected from the group consisting of: nivolumab, pembrolizumab, pidilizumab, PDR001, MEDI0680, TSR-042, REGN2810, JS001, PF-06801591, BGB-A317, BI 754091, and SHR-1210.

22. The method of any of clauses 1-21, wherein the anti-PD-1 antibody comprises CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17.

23. The method of any of clauses 1-21, wherein the anti-PD-1 antibody comprises

(a) a heavy chain variable region CDR1 comprising the sequence set forth in SEQ ID NO:19;
(b) a heavy chain variable region CDR2 comprising the sequence set forth in SEQ ID NO:20;
(c) a heavy chain variable region CDR3 comprising the sequence set forth in SEQ ID NO:21;
(d) a light chain variable region CDR1 comprising the sequence set forth in SEQ ID NO:22;
(e) a light chain variable region CDR2 comprising the sequence set forth in SEQ ID NO:23; and
(f) a light chain variable region CDR3 comprising the sequence set forth in SEQ ID NO:24.

24. The method of any of clauses 1-23, wherein the anti-PD-1 antibody comprises heavy and light chain variable regions comprising the sequences set forth in SEQ ID NOs: 15 and 17, respectively.

25. The method of any of clauses 1-21, wherein the anti-PD-1 antibody comprises heavy and light chains comprising the sequences as set forth in SEQ ID NOs: 13 and 14, respectively.

26. The method of any of clauses 1-25, wherein the one or more chemotherapeutic agents are platinum compounds or fluoropyrimidines.

27. The method of any of clauses 1-26, wherein the one or more chemotherapeutic agents are oxaliplatin, cisplatin, fluorouracil, capecitabine, tegafur, gimeracil, or oteracil.

28. The method of clause 27, wherein the one or more chemotherapeutic agents are oxaliplatin and capecitabine (XELOX).

29. The method of clause 27, wherein the one or more chemotherapeutic agents are oxaliplatin and fluorouracil.

30. The method of clause 27, wherein the chemotherapeutic agents further comprise a chemoprotective agent.

31. The method of clause 30, wherein the chemoprotective agent is leucovorin.

32. The method of clause 31, wherein the one or more chemotherapeutic agents comprise oxaliplatin, leucovorin, and fluorouracil (FOLFOX).

33. The method of clause 27, wherein the one or more chemotherapeutic agents are oxaliplatin and tegafur/gimeracil/oteracil potassium (SOX).

34. The method of any of clauses 1-33, wherein a fixed dose combination of the anti-LAG-3 and anti-PD-1 antibody are administered.

35. The method of clause 34, wherein the fixed dose is determined based on the chemotherapeutic agent administered to the subject.

36. The method of any of clauses 1-35, wherein the method comprises at least one administration cycle, wherein the cycle is a period of six weeks, and wherein for each of the at least one cycle, two doses of the anti-LAG-3 antibody are administered at a dose of 120 or 160 mg and two doses of the anti-PD-1 antibody are administered at a dose of 360 or 480 mg.

37. The method of any of clauses 1-36, wherein 120 mg of the anti-LAG-3 antibody, 360 mg of the anti-PD-1 antibody, and XELOX are administered.

38. The method of any of clauses 1-36, wherein 160 mg of the anti-LAG-3 antibody, 480 mg of the anti-PD-1 antibody, and FOLFOX are administered.

39. The method of any of clauses 1-36, wherein 120 mg of the anti-LAG-3 antibody, 360 mg of the anti-PD-1 antibody, and SOX are administered.

40. The method of any of clauses 1-39, wherein the anti-LAG-3 and anti-PD-1 antibodies are formulated for intravenous administration.

41. The method of any of clauses 1-40, wherein the anti-LAG-3 and anti-PD-1 antibodies are formulated together.

42. The method of any of clauses 1-40, wherein the anti-LAG-3 and anti-PD-1 antibodies are formulated separately.

43. A method of inhibiting the growth of a gastric adenocarcinoma or gastroesophageal junction adenocarcinoma in a human patient, the method comprising administering to the patient an effective amount of each of:

  (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,
  (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and
  (c) one or more chemotherapeutic agents selected from the group consisting of oxaliplatin/capecitabine (XELOX), oxaliplatin/leucovorin/fluorouracil (FOLFOX), and oxaliplatin/tegafur/gimeracil/oteracil (SOX),

wherein the patient's tumor-associated immune cells express LAG-3.

44. A method of treating gastric cancer or gastroesophageal junction cancer in a human patient, the method comprising administering to the patient an effective amount of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,
(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and
(c) one or more chemotherapeutic agents selected from the group consisting of XELOX, FOLFOX, and SOX.

45. The method of clause 44, wherein the patient's tumor-associated immune cells express LAG-3.

46. The method of clause 44 or clause 45, wherein the method is administered to a patient that has not received prior therapy (e.g., first line therapy).

47. The method of clause 46, wherein the prior therapy is administration of a HER2 inhibitor.

48. The method of any one of clauses 44-47, wherein the method is administered to a patient who is HER2 negative.

49. The method of any one of clauses 44-48, wherein the patient has not received any prior systematic treatment.

50. The method of any one of clauses 44-49, wherein the anti-LAG-3 antibody and the anti-PD-1 antibody are administered as a fixed dose combination.

51. The method of any one of clauses 44-50, wherein the gastric cancer or gastroesophageal junction cancer is recurrent, locally advanced or metastatic gastric cancer or gastoesophageal adenocarcinoma.

52. The method of any of clauses 1-51, wherein expression of LAG-3 is assayed by RT-PCR, *in situ* hybridization, RNase protection, RT-PCR-based assay, immunohistochemistry, enzyme linked immuosorbent assay, *in vivo* imaging, or flow cytometry.

53. The method of clause 52, wherein LAG-3 expression is assayed by immunohistochemistry.

54. A method of treating gastric cancer or gastroesophageal junction adenocarcinoma in a human patient, the method comprising administering to the patient an effective amount of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,
(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and
(c) one or more chemotherapeutic agents.

55. The method of clause 54, wherein the method is administered to a patient that has not received prior therapy (e.g., first line therapy).

56. A method of treating recurrent, locally advanced or metastatic gastric cancer or gastroesophageal junction adenocarcinoma in a human patient, the method comprising administering to the patient an effective amount of :

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and
(b) one or more standard-of-care therapeutic regimens,

wherein the patient's tumor-associated immune cells express LAG-3.

57. A method of treating recurrent, locally advanced or metastatic gastric cancer or gastroesophageal junction adenocarcinoma in a human patient, the method comprising administering to the patient an effective amount of :

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and
(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and
(c) one or more standard-of-care therapeutic regimens,

wherein the patient's tumor-associated immune cells express LAG-3.

58. A method of treating recurrent, locally advanced or metastatic gastric cancer or gastroesophageal junction adenocarcinoma in a human patient, the method comprising administering to the patient an effective amount of :

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and
(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17, and
(c) one or more standard-of-care therapeutic regimens.

59. The method of clause 57 or clause 58, wherein the anti-LAG-3 and anti-PD-1 antibodies are administered as a fixed dose combination.

60. The method of any one of clauses 57-59, wherein the one or more standard-of-care therapeutic regimens comprises administration of docetaxel, doxorubicin hydrochloride, 5-fluorouracil, mitomycin C, fluorouracil/leucovorin calcium (FU-LV), docetaxel/cisplatin/fluorouracil (TPF), or capecitabine/irinotecan hydrochloride (XELIRI).

61. The method of any of clauses 57-60, wherein the method is administered to a patient that has received a prior therapy (e.g., as a second line therapy).

62. The method of any one of clauses 1-61, wherein the anti-LAG-3 antibody is Relatlimab.

63. The method of any one of clauses 1-62, wherein the anti-LAG-3 antibody comprises a serine to proline mutation at amino acid residue 228.

**Claims**

1.  A LAG-3 antagonist for use in treating cancer in a human patient in combination with a PD-1 pathway inhibitor and one or more chemotherapeutic agents.

2.  The LAG-3 antagonist for use of claim 1, wherein the patient's tumor associated immune cells express LAG-3.

3.  The LAG-3 antagonist for use of claim 1 or 2, wherein the cancer is:

(a) a gastric cancer, gastroesophageal junction cancer, liver cancer, bone cancer, pancreatic cancer, skin cancer, oral cancer, cancer of the head or neck, breast cancer, lung cancer, melanoma, renal cancer, uterine cancer, ovarian cancer, colorectal cancer, colon cancer, rectal cancer, cancer of the anal region, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, cancers of the childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancer, hematologic malignancy, or any combination thereof,

(b) a gastric cancer that is an adenocarcinoma, lymphoma, gastrointestinal stromal tumor, carcinoid tumor, or any combination thereof, or

(c) a cutaneous or intraocular malignant melanoma, small cell lung cancer, non-small cell lung cancer (NSCLC), human papilloma virus (HPV)-related tumor, multiple myeloma, B-cell lymphoma, Hodgkin lymphoma/primary mediastinal B-cell lymphoma, non-Hodgkin's lymphoma, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, precursor T-lymphoblastic lymphoma, head and neck squamous cell carcinoma, renal cell cancer, hepatocellular carcinoma, or any combination thereof.

4. The LAG-3 antagonist for use of any one of claims 1-3, wherein the LAG-3 antagonist is an anti-LAG-3 antibody or a soluble LAG-3 polypeptide.

5. The LAG-3 antagonist for use of claim 4, wherein the anti-LAG-3 antibody is a full-length antibody.

6. The LAG-3 antagonist for use of claim 5, wherein the anti-LAG-3 antibody is a monoclonal, human, humanized, chimeric, or multispecific antibody.

7. The LAG-3 antagonist for use of claim 6, wherein the multispecific antibody is a dual-affinity re-targeting antibody (DART), a DVD-Ig, or bispecific antibody.

8. The LAG-3 antagonist for use of claim 4, wherein the anti-LAG-3 antibody is a F(ab')$_2$ fragment, a Fab' fragment, a Fab fragment, a Fv fragment, a scFv fragment, a dsFv fragment, a dAb fragment, or a single chain binding polypeptide.

9. The LAG-3 antagonist for use of claim 4, wherein the anti-LAG-3 antibody comprises:

(a) BMS-986016, IMP731 (H5L7BW), MK-4280 (28G-10), REGN3767, GSK2831781, humanized BAP050, IMP-701 (LAG-5250), aLAG3(0414), aLAG3(0416), Sym022, TSR-033, TSR-075, XmAb22841, BI 754111, MGD013, AVA-017, P 13B02-30, or FS-118,
(b) CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,
(c) CDR1, CDR2 and CDR3 domains of the heavy chain variable region having the sequences set forth in SEQ ID NOs:7, 8, and 9, respectively, and CDR1, CDR2 and CDR3 domains of the light chain variable region having the sequences set forth in SEQ ID NOs:10, 11, and 12, respectively,
(d) heavy and light chain variable regions comprising the sequences set forth in SEQ ID NOs:3 and 5, respectively, or
(e) heavy and light chains comprising the sequences set forth in SEQ ID NOs: 1 and 2, respectively.

10. The LAG-3 antagonist for use of claim 4, wherein the soluble LAG-3 polypeptide comprises IMP321 (eftilagimod alpha).

11. The LAG-3 antagonist for use of any one of claims 1-10, wherein the PD-1 pathway inhibitor is an anti-PD-1 or an anti-PD-L1 antibody.

12. The LAG-3 antagonist for use of claim 11, wherein the anti-PD-1 antibody comprises:

(a) nivolumab, pembrolizumab, pidilizumab, PDR001, MEDI0680, TSR-042, REGN2810, JS001, PF-06801591, BGB-A317, BI 754091, and SHR-1210,
(b) CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17,
(c) CDR1, CDR2 and CDR3 domains of the heavy chain variable region having the sequences set forth in SEQ ID NOs:19, 20, and 21, respectively, and CDR1, CDR2 and CDR3 domains of the light chain variable region having the sequences set forth in SEQ ID NOs:22, 23, and 24, respectively,

(d) heavy and light chain variable regions comprising the sequences set forth in SEQ ID NOs: 15 and 17, respectively, or

(e) heavy and light chains comprising the sequences as set forth in SEQ ID NOs: 13 and 14, respectively.

13. The LAG-3 antagonist for use of any one of claims 1-9 or 11-12, wherein the LAG-3 antagonist is anti-LAG-3 antibody and the PD-1 pathway inhibitor is an anti-PD-1 antibody, and wherein:

(a) the anti-LAG-3 antibody comprises:

(i) BMS-986016, IMP731 (H5L7BW), MK-4280 (28G-10), REGN3767, GSK2831781, humanized BAP050, IMP-701 (LAG-5250), aLAG3(0414), aLAG3(0416), Sym022, TSR-033, TSR-075, XmAb22841, BI 754111, MGD013, AVA-017, P 13B02-30, or FS-118,

(ii) CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,

(iii) CDR1, CDR2 and CDR3 domains of the heavy chain variable region having the sequences set forth in SEQ ID NOs:7, 8, and 9, respectively, and CDR1, CDR2 and CDR3 domains of the light chain variable region having the sequences set forth in SEQ ID NOs:10, 11, and 12, respectively,

(iv) heavy and light chain variable regions comprising the sequences set forth in SEQ ID NOs:3 and 5, respectively, or

(v) heavy and light chains comprising the sequences set forth in SEQ ID NOs:1 and 2, respectively, and

(b) the anti-PD-1 antibody comprises:

(i) nivolumab, pembrolizumab, pidilizumab, PDR001, MEDI0680, TSR-042, REGN2810, JS001, PF-06801591, BGB-A317, BI 754091, or SHR-1210,

(ii) CDR1, CDR2 and CDR3 domains of the heavy chain variable region having the sequence set forth in SEQ ID NO:15, and CDR1, CDR2 and CDR3 domains of the light chain variable region having the sequence set forth in SEQ ID NO:17,

(iii) CDR1, CDR2 and CDR3 domains of the heavy chain variable region having the sequences set forth in SEQ ID NOs:19, 20, and 21, respectively, and CDR1, CDR2 and CDR3 domains of the light chain variable region having the sequences set forth in SEQ ID NOs:22, 23, and 24, respectively,

(iv) heavy and light chain variable regions comprising the sequences set forth in SEQ ID NOs:15 and 17, respectively, or

(v) heavy and light chains comprising the sequences set forth in SEQ ID NOs:13 and 14, respectively

14. The LAG-3 antagonist for use of any one of claims 1-13, wherein the one or more chemotherapeutic agents comprise:

(a) platinum compounds or fluoropyrimidines,

(b) oxaliplatin, cisplatin, fluorouracil, capecitabine, tegafur, gimeracil, or oteracil,

(c) oxaliplatin and capecitabine (XELOX),

(d) oxaliplatin and fluorouracil,

(e) oxaliplatin, cisplatin, fluorouracil, capecitabine, tegafur, gimeracil, or oteracil, and a chemoprotective agent,

(f) oxaliplatin, cisplatin, fluorouracil, capecitabine, tegafur, gimeracil, or oteracil, and leucovorin,

(g) oxaliplatin, leucovorin, and fluorouracil (FOLFOX), or

(h) oxaliplatin and tegafur/gimeracil/oteracil potassium (SOX).

15. The LAG-3 antagonist for use of claim 13 or 14, wherein a fixed dose combination of the anti-LAG-3 and anti-PD-1 antibody are administered.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62703690 A
- US 62725336 B
- US 8008449 B
- US 7943743 B
- US 2016029878 W
- US 1348999 W
- US 20110150892 A1
- US 2011007023 A
- WO 2016028672 A
- WO 2017019894 A
- US 10188730 B
- WO 2017106129 A
- WO 2017062888 A
- WO 2009044273 A
- WO 2018069500 A
- WO 2016126858 A
- WO 2014179664 A
- WO 2016200782 A
- WO 2015200119 A
- WO 2017019846 A
- WO 2017198741 A
- WO 2017220555 A
- WO 2017220569 A
- WO 2018071500 A
- WO 2017015560 A
- WO 2017025498 A
- WO 2017087589 A
- WO 2017087901 A
- WO 2018083087 A
- WO 2017149143 A
- WO 2017219995 A
- US 20170260271 A
- WO 2017086367 A
- WO 2017086419 A
- WO 2018034227 A
- WO 18185046 A
- WO 18185043 A
- WO 2018217940 A
- WO 19011306 A
- WO 2018208868 A
- WO 2014140180 A
- WO 2006121168 A
- WO 2008156712 A
- WO 2012145493 A
- WO 2009014708 A
- WO 03099196 A
- WO 2009114335 A
- WO 2011161699 A
- US 8779105 B
- US 6808710 B
- US 7488802 B
- US 8168757 B
- US 8354509 B
- US 8900587 B
- US 8609089 B2
- US 20130017199 A
- US 20150079109 A
- US 8686119 B2
- WO 2013014668 A1
- US 20160272708 A
- WO 2015112900 A
- WO 2015112800 A
- WO 2014206107 A
- WO 201535606 A
- WO 2015085847 A
- WO 2017020291 A
- WO 2017020858 A
- WO 2016197367 A
- WO 2017024515 A
- WO 2017025051 A
- WO 2017123557 A
- WO 2016106159 A
- WO 2014194302 A
- WO 2017040790 A
- WO 2017133540 A
- WO 2017132827 A
- WO 2017024465 A
- WO 2017025016 A
- WO 2017106061 A
- WO 201719846 A
- WO 2017132825 A
- WO 2013173223 A
- US 8217149 B
- US 7635757 B
- US 20090317368 A
- WO 2011066389 A
- US 9580507 B
- US 20140341917 A
- WO 2013079174 A
- WO 2013181634 A
- WO 2016149201 A
- WO 2017034916 A, Eli Lilly Co.

**Non-patent literature cited in the description**

- **TRIEBEL F et al.** *J. Exp. Med.*, 1990, vol. 171, 1393-1405
- **WORKMAN C J et al.** *J. Immunol.*, 2009, vol. 182 (4), 1885-91
- **KEIR M E et al.** *Annu Rev Immunol*, 2008, vol. 26, 677-704
- **DONG H et al.** *Nat Med.*, 1999, vol. 5, 1365-1369
- **TERME M et al.** *Cancer Res*, 2011, vol. 71, 5393-5399
- **PARDOLL D M.** *Nat Rev Cancer*, 2012, vol. 12, 252-264
- **NISHIMURA H et al.** *Immunity*, 1999, vol. 11, 141-151
- **NISHIMURA H et al.** *Science*, 2001, vol. 291, 319-322
- **DONG H et al.** *Nat Med*, 2002, vol. 8, 793-800
- **TOPALIAN S L et al.** *NEJM*, 2012, vol. 366 (26), 2443-2454
- **HAMID O et al.** *NEJM*, 2013, vol. 369, 134-144
- **WARD et al.** *Nature*, 1989, vol. 341, 544-46
- **BIRD et al.** *Science*, 1988, vol. 242, 423-426
- **HUSTON et al.** *Proc. Natl. Acad. Sci. USA*, 1988, vol. 85, 5879-5883
- **OKAZAKI et al.** *Curr. Opin. Immunol.*, 2002, vol. 14, 391779-82
- **BENNETT et al.** *J Immunol*, 2003, vol. 170, 711-8
- **HUTLOFF et al.** *Nature*, 1999, vol. 397, 263-266
- **HANSEN et al.** *Immunogenics*, 1980, vol. 10, 247-260
- **ISHIDA et al.** *EMBO J*, 1992, vol. 11, 3887-95
- **AGATA et al.** *Int Immunol*, 1996, vol. 8, 765-72
- **THOMAS, M. L.** *J Exp Med*, 1995, vol. 181, 1953-6
- **VIVIER, E** ; **DAERON, M**. *Immunol Today*, 1997, vol. 18, 286-91
- **FREEMAN et al.** *J Exp Med*, vol. 192, 1027-34
- **LATCHMAN et al.** *Nat Immunol*, 2001, vol. 2, 261-8
- **CARTER et al.** *Eur J Immunol*, 2002, vol. 32, 634-43
- **DONG et al.** *Nat. Med.*, 2002, vol. 8, 787-9
- **DONG et al.** *J. Mol. Med.*, 2003, vol. 81, 281-7
- **BLANK et al.** *Cancer Immunol. Immunother.*, 2005, vol. 54, 307-314

- **KONISHI et al.** *Clin. Cancer Res.*, 2004, vol. 10, 5094-100
- **IWAI et al.** *Proc. Nat'l. Acad. Sci. USA*, 2002, vol. 99, 12293-7
- **BROWN et al.** *J. Immunol.*, 2003, vol. 170, 1257-66
- **NISHIMURA et al.** *Immunity*, 1999, vol. 11, 141-51
- **NISHIMURA et al.** *Science*, 2001, vol. 291, 319-22
- **SALAMA et al.** *J Exp Med*, 2003, vol. 198, 71-78
- **PROKUNINA** ; **ALARCON-RIQUELME**. *Hum Mol Genet*, 2004, vol. 13, R143
- **NIELSEN et al.** *Lupus*, 2004, vol. 13, 510
- **OKAZAKI et al.** *PNAS*, 2001, vol. 98, 13866-71
- Concise Dictionary of Biomedicine and Molecular Biology, Juo, Pei-Show. CRC Press, 2002
- The Dictionary of Cell and Molecular Biology. Academic Press, 2013
- Oxford Dictionary Of Biochemistry And Molecular Biology. Oxford University Press, 2006
- **CONDEELIS** ; **WEISSLEDER**. *Cold Spring Harb. Perspect. Biol.*, 2010, vol. 2 (12), a003848
- **MCCABE** ; **WU**. *Cancer Biother. Radiopharm.*, 2010, vol. 25 (3), 253-61
- **OLAFSEN et al.** *Protein Eng. Des. Sel.*, 2010, vol. 23 (4), 243-9
- *Journal for ImmunoTherapy of Cancer*, 2016, vol. 4 (1), 195
- **J. MATSUZAKI et al.** *PNAS*, 2010, vol. 107, 7875
- **WANG et al.** *Cancer Immunol Res.*, 2014, vol. 2 (9), 846-56
- **SI-YANG LIU et al.** *J. Hematol. Oncol.*, 2017, vol. 10, 136
- **HERBST et al.** *J Clin Oncol*, 2013, vol. 31, 3000
- **KHLEIF**. *Proceedings from the European Cancer Congress*, 27 September 2013, 802
- **KHLEIF**. *Proceedings from the European Cancer Congress 2013*, 27 September 2013, 802
- **ZHANG et al.** *Cell Discov.*, March 2017, vol. 7, 3
- **GORELIK et al.** *AACR*, April 2016, 4606
- **NICOLAOU et al.** *Angew. Chem Intl. Ed. Engl.*, 1994, vol. 33, 183-186